# EUROPEAN PATENT SPECIFICATION

(11) **EP 3 187 151 B1**
(45) Date of publication and mention of the grant of the patent: **05.12.2018**
(21) Application number: 16188364.0
(22) Date of filing: 13.04.2013
(51) Int. Cl.: A61F 2/30, A61B 17/15, A61F 2/38

(54) **PATIENT ADAPTED JOINT ARTHROPLASTY DEVICES AND SURGICAL TOOLS**
PATIENTENADAPTIERTE GELENKARTHROPLASTIEVORRICHTUNGEN UND CHIRURGISCHE INSTRUMENTE
DISPOSITIFS D'ARTHROPLASTIE D'ARTICULATION ADAPTÉS AU PATIENT ET OUTILS CHIRURGICAUX

(30) Priority: 13.04.2012 US 201261624230 P
(43) Date of publication of application: 05.07.2017
(62) Divisional of application: 13724036.2
(73) Proprietor: ConforMIS, Inc., Billerica, MA 01821 (US)
(72) Inventor: LANG, Philipp, Lexington, MA 02421 (US); STEINES, Daniel, Lexington, MA 02421 (US)
(74) Representative: Grund, Martin

(56) References cited:
- WO-A1-2010/121147
- US-A1- 2003 216 669
- US-A1- 2009 226 068
- US-A1- 2012 041 446

## Description

### FIELD

This disclosure relates to systems for joint arthroplasty including articular resurfacing. The closest prior art is document US 2012/0041446 A1, which defines the preamble of claim 1.

### BACKGROUND

Usually, severe damage or loss of articular cartilage is treated by replacement of the joint with a prosthetic material, for example, silicone, *e.g.* for cosmetic repairs, or metal alloys. Joint arthroplasties are highly invasive and require surgical resection of the entire or the majority of the articular surface of one or more bones. For example, in certain procedures, the marrow space is reamed in order to fit the stem of the prosthesis. Reaming results in a loss of the patient's bone stock and over time subsequent osteolysis will frequently lead to loosening of the prosthesis. Further, the area where the implant and the bone mate degrades over time requiring the prosthesis to eventually be replaced. Since the patient's bone stock is limited, the number of possible replacement surgeries is also limited for joint arthroplasty. In short, over the course of 15 to 20 years, and in some cases even shorter time periods, the patient could run out of therapeutic options ultimately resulting in a painful, non-functional joint.

Thus, there remains a need for compositions for joint repair. There is also a need for tools that increase the accuracy of cuts made to the bone in a joint in preparation for surgical implantation of, for example, an artificial joint.

### SUMMARY

Various exemplary embodiments include a system for performing a joint arthroplasty procedure at a surgical site. Such a system can include an implant and a patient-adapted surgical tool. The system can also include measurement devices configured for use in obtaining one or more kinematic measurements. Further, the system can include adjustment tools configured for use during the joint arthroplasty procedure to optimize the procedure based, at least in part, on the kinematic measurements.

### BRIEF DESCRIPTION OF THE DRAWINGS

The foregoing features of the invention will be more readily understood by reference to the following detailed description, taken with reference to the accompanying drawings, in which:
**FIG. 21A** illustrates a femur, tibia and fibula along with the mechanical and anatomic axes. **FIGS. 21B-E** illustrate the tibia with the anatomic and mechanical axis used to create a cutting plane along with a cut femur and tibia. **FIG. 21F** illustrates the proximal end of the femur including the head of the femur.
**FIG. 22** shows an example of a surgical tool having one surface matching the geometry of an articular surface of the joint, in accordance with one embodiment. Also shown is an aperture in the tool capable of controlling drill depth and width of the hole and allowing implantation of an insertion of implant having a press-fit design.
**FIG. 23** is a flow chart depicting various methods of the invention used to create a mold for preparing a patient's joint for arthroscopic surgery.

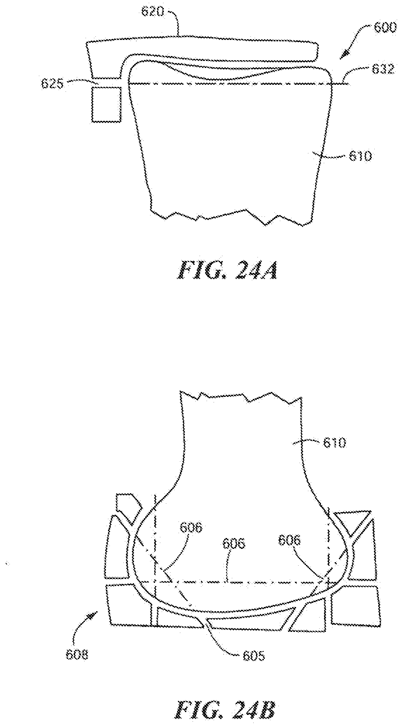

**FIG. 24A** depicts, in cross-section, an example of a surgical tool containing an aperture through which a surgical drill or saw can fit, in accordance with one embodiment. The aperture guides the drill or saw to make the proper hole or cut in the underlying bone. Dotted lines represent where the cut corresponding to the aperture will be made in bone. **FIG. 24B** depicts, in cross-section, an example of a surgical tool containing apertures through which a surgical drill or saw can fit and which guide the drill or saw to make cuts or holes in the bone, in accordance with one embodiment. Dotted lines represent where the cuts corresponding to the apertures will be made in bone.
**FIGS. 25A-R** illustrate tibial cutting blocks and molds used to create a surface perpendicular to the anatomic axis for receiving the tibial portion of a knee implant, in accordance with various embodiments.
**FIGS. 26A-O** illustrate femur cutting blocks and molds used to create a surface for receiving the femoral portion of a knee implant, in accordance with various embodiments.
**FIG. 28A-H** illustrate femoral head cutting blocks and molds used to create a surface for receiving the femoral portion of a knee implant, in accordance with various embodiments.
**FIG. 29A-D** illustrate acetabulum cutting blocks and molds used to create a surface for a hip implant, in accordance with various embodiments.
**FIG. 30** illustrates a 3D guidance template in a hip joint, wherein the surface of the template facing the joint is a mirror image of a portion of the joint that is not affected by the arthritic process, in accordance with one embodiment.
**FIG. 31** illustrates a 3D guidance template for an acetabulum, wherein the surface of the template facing the joint is a mirror image of a portion of the joint that is affected by the arthritic process, in accordance with one embodiment.

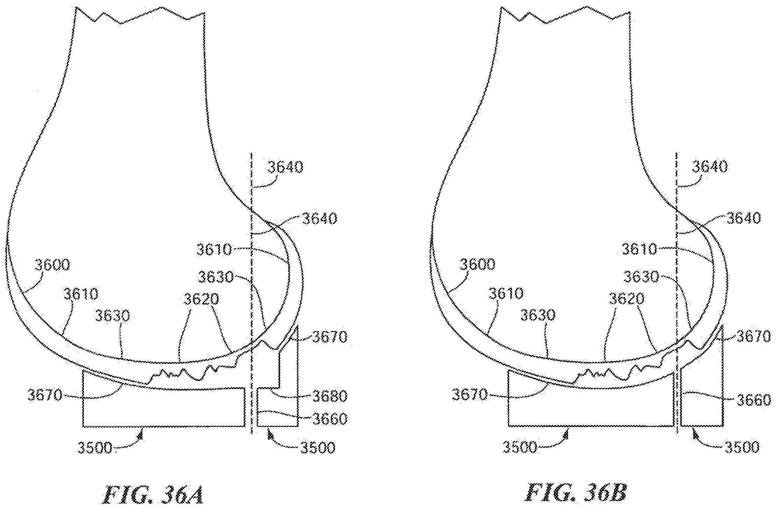

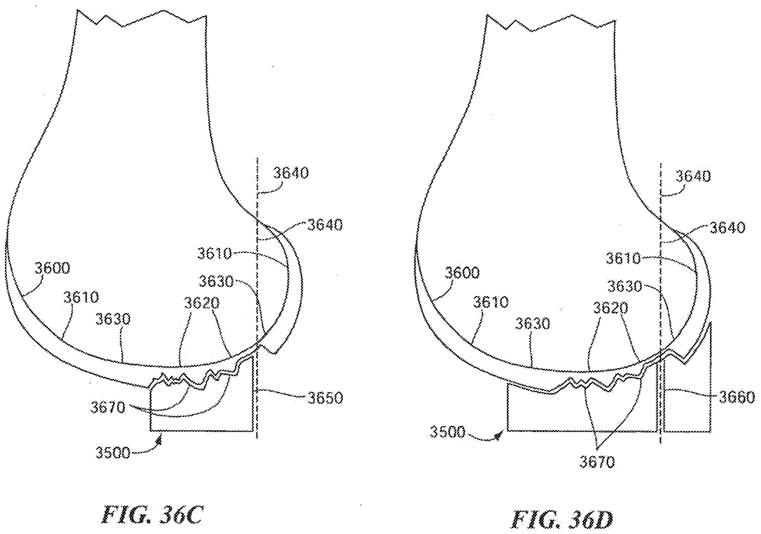

**FIG. 36A** illustrates a 3D guidance template wherein the surface of the template facing the joint is a mirror image of at least portions of the surface of a joint that is healthy or substantially unaffected by the arthritic process, in accordance with one embodiment. **FIG. 36B** illustrates the 3D guidance template wherein the surface of the template facing the joint is a mirror image of at least portions of the surface of the joint that is healthy or substantially unaffected by the arthritic process, in accordance with one embodiment. The diseased area is covered by the template, but the mold is not substantially in contact with it. **FIG. 36C** illustrates the 3D guidance template wherein the surface of the template facing the joint is a mirror image of at least portions of the surface of the joint that are arthritic, in accordance with one embodiment. **FIG. 36D** illustrates the 3D guidance template wherein the template closely mirrors the shape of the interface between substantially normal or near normal and diseased joint tissue, in accordance with one embodiment.
**FIGS. 37A-D** show multiple molds with linkages on the same articular surface (A-C) and to an opposing articular surface (D), in accordance with various embodiments.
**FIG. 39** is a flow diagram showing a method wherein measured leg length discrepancy is utilized to determine the optimal cut height of a femoral neck cut for total hip arthroplasty, in accordance with one embodiment.
**FIGS. 40A-C** illustrate the use of 3D guidance templates for performing ligament repair, in accordance with one embodiment.
**FIG. 43** shows an example of an intended site for placement of a femoral neck mold for total hip arthroplasty, in accordance with one embodiment.
**FIG. 44** shows an example of a femoral neck mold with handle and slot, in accordance with one embodiment.
**FIG. 46** shows an example of a guidance mold used for reaming the site for an acetabular cup, in accordance with one embodiment.
**FIG. 47** shows an example of an optional second femoral neck mold, placed on the femoral neck cut, providing and estimate of anteversion and longitudinal femoral axis.

### DETAILED DESCRIPTION

Various modifications to the embodiments described will be readily apparent to those skilled in the art, and the generic principles defined herein can be applied to other embodiments and applications without departing from the scope of the disclosure. Thus, the present invention is not intended to be limited to the embodiments shown, but is to be accorded the widest scope consistent with the the scope of the claims appended hereto.

3D guidance surgical tools, referred to herein as a 3D guidance surgical templates, that may be used for surgical assistance may include, without limitation, using templates, jigs and/or molds, including 3D guidance molds. It is to be understood that the terms "template," "jig," "mold," "3D guidance mold," and "3D guidance template," shall be used interchangeably within the detailed description and appended claims to describe the tool unless the context indicates otherwise.

3D guidance surgical tools that may be used may include guide apertures. It is to be understood that the term guide aperture shall be used interchangeably within the detailed description and appended claims to describe both guide surface and guide elements.

As will be appreciated by those of skill in the art, the practice of the present invention employs, unless otherwise indicated, conventional methods of x-ray imaging and processing, x-ray tomosynthesis, ultrasound including A-scan, B-scan and C-scan, computed tomography (CT scan), magnetic resonance imaging (MRI), optical coherence tomography, single photon emission tomography (SPECT) and positron emission tomography (PET) within the skill of the art. Such techniques are explained fully in the literature and need not be described herein. See, *e.g.,* X-Ray Structure Determination: A Practical Guide, 2nd Edition, editors Stout and Jensen, 1989, John Wiley & Sons, publisher; Body CT: A Practical Approach, editor Slone, 1999, McGraw-Hill publisher; X-ray Diagnosis: A Physician's Approach, editor Lam, 1998 Springer-Verlag, publisher; and Dental Radiology: Understanding the X-Ray Image, editor Laetitia Brocklebank 1997, Oxford University Press publisher. *See also,* The Essential Physics of Medical Imaging (2nd Ed.), Jerrold T. Bushberg, et al.

This disclosure provides methods and compositions for repairing joints, particularly for repairing articular cartilage and for facilitating the integration of a wide variety of cartilage repair materials into a subject. Among other things, the techniques described herein allow for the customization of cartilage repair material to suit a particular subject, for example in terms of size, cartilage thickness and/or curvature. When the shape (*e.g.,* size, thickness and/or curvature) of the articular cartilage surface is an exact or near anatomic fit with the non-damaged cartilage or with the subject's original cartilage, the success of repair is enhanced. The repair material can be shaped prior to implantation and such shaping can be based, for example, on electronic images that provide information regarding curvature or thickness of any "normal" cartilage surrounding the defect and/or on curvature of the bone underlying the defect. Thus, this disclosure provides, among other things, for minimally invasive methods for partial joint replacement. The methods will require only minimal or, in some instances, no loss in bone stock. Additionally, unlike with current techniques, the methods described herein will help to restore the integrity of the articular surface by achieving an exact or near anatomic match between the implant and the surrounding or adjacent cartilage and/or subchondral bone.

### A. MEASUREMENT TECHNIQUES

As will be appreciated by those of skill in the art, imaging techniques suitable for measuring thickness and/or curvature (*e.g.,* of cartilage and/or bone) or size of areas of diseased cartilage or cartilage loss include the use of x-rays, magnetic resonance imaging (MRI), computed tomography scanning (CT, also known as computerized axial tomography or CAT), optical coherence tomography, ultrasound imaging techniques, and optical imaging techniques. (*See,* also, International Patent Publication WO 02/22014 to Alexander, et al., published March 21, 2002; U.S. Patent No. 6,373,250 to Tsoref et al., issued April 16, 2002; and Vandeberg et al. (2002) Radiology 222:430-436). Contrast or other enhancing agents can be employed using any route of administration, e.g. intravenous, intra-articular, etc.

Alternatively, or in addition to, various other non-invasive imaging techniques, measurements of the size of an area of diseased cartilage or an area of cartilage loss, measurements of cartilage thickness and/or curvature of cartilage or bone can be obtained intraoperatively during arthroscopy or open arthrotomy. Intraoperative measurements can, but need not, involve actual contact with one or more areas of the articular surfaces.

Devices suitable for obtaining intraoperative measurements of cartilage or bone or other articular structures, and to generate a topographical map of the surface include but are not limited to, Placido disks, optical measurements tools and device, optical imaging tools and devices, and laser interferometers, and/or deformable materials or devices. (See, for example, U.S. Patent Numbers 6,382,028 to Wooh et al., issued May 7, 2002; 6,057,927 to Levesque et al., issued May 2, 2000; 5,523,843 to Yamane et al. issued June 4, 1996; 5,847,804 to Sarver et al. issued December 8, 1998; and 5,684,562 to Fujieda, issued November 4, 1997).

Mechanical devices (*e.g.,* probes) can also be used for intraoperative measurements, for example, deformable materials such as gels, molds, any hardening materials (*e.g.,* materials that remain deformable until they are heated, cooled, or otherwise manipulated). See, *e.g.,* WO 02/34310 to Dickson et al., published May 2, 2002. For example, a deformable gel can be applied to a femoral condyle. The side of the gel pointing towards the condyle can yield a negative impression of the surface contour of the condyle. The negative impression can then be used to determine the size of a defect, the depth of a defect and the curvature of the articular surface in and adjacent to a defect. This information can be used to select a therapy, *e.g.* an articular surface repair system or a mold. It can also be used to make a mold, either directly with use of the impression or, for example, indirectly via scanning the impression. In another example, a hardening material can be applied to an articular surface, *e.g.* a femoral condyle or a tibial plateau. The hardening material can remain on the articular surface until hardening has occurred. The hardening material can then be removed from the articular surface. The side of the hardening material pointing towards the articular surface can yield a negative impression of the articular surface. The negative impression can then be used to determine the size of a defect, the depth of a defect and the curvature of the articular surface in and adjacent to a defect. This information can then be used to select a therapy, *e.g.* an articular surface repair system or a mold. It can also be used to make a mold, either directly with use of the impression or, for example, indirectly via scanning the impression. In some embodiments, the hardening system can remain in place and form the actual articular surface repair system.

In certain embodiments, the deformable material comprises a plurality of individually moveable mechanical elements. When pressed against the surface of interest, each element can be pushed in the opposing direction and the extent to which it is pushed (deformed) can correspond to the curvature of the surface of interest. The device can include a brake mechanism so that the elements are maintained in the position that conforms to the surface of the cartilage and/or bone. The device can then be removed from the patient and analyzed for curvature. Alternatively, each individual moveable element can include markers indicating the amount and/or degree it is deformed at a given spot. A camera can be used to intra-operatively image the device and the image can be saved and analyzed for curvature information. Suitable markers include, but are not limited to, actual linear measurements (metric or empirical), different colors corresponding to different amounts of deformation and/or different shades or hues of the same color(s). Displacement of the moveable elements can also be measured using electronic means.

Other devices to measure cartilage and subchondral bone intraoperatively include, for example, ultrasound probes. An ultrasound probe, preferably handheld, can be applied to the cartilage and the curvature of the cartilage and/or the subchondral bone can be measured. Moreover, the size of a cartilage defect can be assessed and the thickness of the articular cartilage can be determined. Such ultrasound measurements can be obtained in A-mode, B-mode, or C-mode. If A-mode measurements are obtained, an operator can typically repeat the measurements with several different probe orientations, *e.g.* mediolateral and anteroposterior, in order to derive a three-dimensional assessment of size, curvature and thickness.

One skilled in the art will easily recognize that different probe designs are possible using the optical, laser interferometry, mechanical and ultrasound probes. The probes are preferably handheld. In certain embodiments, the probes or at least a portion of the probe, typically the portion that is in contact with the tissue, can be sterile. Sterility can be achieved with use of sterile covers, for example similar to those disclosed in WO 99/08598A1 to Lang, published February 25, 1999.

Analysis on the curvature of the articular cartilage or subchondral bone using imaging tests and/or intraoperative measurements can be used to determine the size of an area of diseased cartilage or cartilage loss. For example, the curvature can change abruptly in areas of cartilage loss. Such abrupt or sudden changes in curvature can be used to detect the boundaries of diseased cartilage or cartilage defects.

As described above, measurements can be made while the joint is stationary, either weight bearing or not, or in motion.

### B. THE JOINT REPLACEMENT PROCEDURE

### i. Knee Joint

Performing a total knee arthroplasty is a complicated procedure. In replacing the knee with an artificial knee, it is important to get the anatomical and mechanical axes of the lower extremity aligned correctly to ensure optimal functioning of the implanted knee.

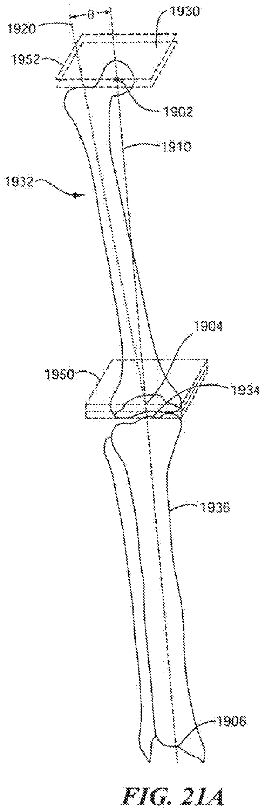

As shown in **FIG. 21A****,** the center of the hip ***1902*** (located at the head ***1930*** of the femur ***1932***), the center of the knee ***1904*** (located at the notch where the intercondylar tubercle ***1934*** of the tibia ***1936*** meet the femur) and ankle ***1906*** lie approximately in a straight line ***1910*** which defines the mechanical axis of the lower extremity. The anatomic axis ***1920*** aligns 5-7° offset ***θ*** from the mechanical axis in the valgus, or outward, direction.

The long axis of the tibia ***1936*** is collinear with the mechanical axis of the lower extremity ***1910.*** From a three-dimensional perspective, the lower extremity of the body ideally functions within a single plane known as the median anterior-posterior plane (MAP-plane) throughout the flexion-extension arc. In order to accomplish this, the femoral head ***1930,*** the mechanical axis of the femur, the patellar groove, the intercondylar notch, the patellar articular crest, the tibia and the ankle remain within the MAP-plane during the flexion-extension movement. During movement, the tibia rotates as the knee flexes and extends in the epicondylar axis which is perpendicular to the MAP-plane.

A variety of image slices can be taken at each individual joint, e.g., the knee joint ***1950-1950ₙ,*** and the hip joint ***1952-1950ₙ.*** These image slices can be used as described above in Section I along with an image of the full leg to ascertain the axis.

With disease and malfunction of the knee, alignment of the anatomic axis is altered. Performing a total knee arthroplasty is one solution for correcting a diseased knee. Implanting a total knee joint, such as the PFC Sigma RP Knee System by Johnson & Johnson, requires that a series of resections be made to the surfaces forming the knee joint in order to facilitate installation of the artificial knee. The resections should be made to enable the installed artificial knee to achieve flexion-extension movement within the MAP-plane and to optimize the patient's anatomical and mechanical axis of the lower extremity.

First, the tibia ***1930*** is resected to create a flat surface to accept the tibial component of the implant. In most cases, the tibial surface is resected perpendicular to the long axis of the tibia in the coronal plane, but is typically sloped 4-7° posteriorly in the sagittal plane to match the normal slope of the tibia. As will be appreciated by those of skill in the art, the sagittal slope can be 0° where the device to be implanted does not require a sloped tibial cut. The resection line ***1958*** is perpendicular to the mechanical axis ***1910,*** but the angle between the resection line and the surface plane of the plateau ***1960*** varies depending on the amount of damage to the knee.

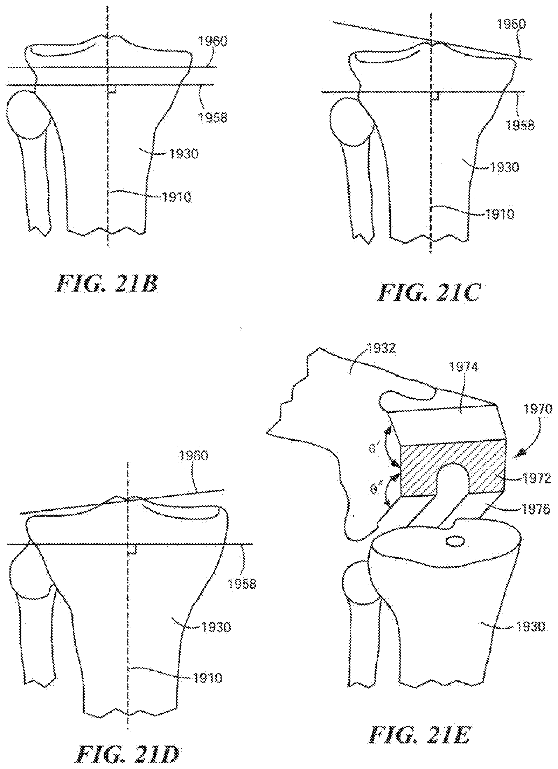

**FIGS. 21B-D** illustrate an anterior view of a resection of an anatomically normal tibial component, a tibial component in a varus knee, and a tibial component in a valgus knee, respectively. In each figure, the mechanical axis ***1910*** extends vertically through the bone and the resection line ***1958*** is perpendicular to the mechanical axis ***1910*** in the coronal plane, varying from the surface line formed by the joint depending on the amount of damage to the joint. **FIG. 21B** illustrates a normal knee wherein the line corresponding to the surface of the joint ***1960*** is parallel to the resection line ***1958.*** **FIG. 21C** illustrates a varus knee wherein the line corresponding to the surface of the joint ***1960*** is not parallel to the resection line ***1958.*** **FIG. 21D** illustrates a valgus knee wherein the line corresponding to the surface of the joint ***1960*** is not parallel to the resection line ***1958.***

Once the tibial surface has been prepared, the surgeon turns to preparing the femoral condyle.

The plateau of the femur ***1970*** is resected to provide flat surfaces that communicate with the interior of the femoral prosthesis. The cuts made to the femur are based on the overall height of the gap to be created between the tibia and the femur. Typically, a 20 mm gap is desirable to provide the implanted prosthesis adequate room to achieve full range of motion. The bone is resected at a 5-7° angle valgus to the mechanical axis of the femur. Resected surface ***1972*** forms a flat plane with an angular relationship to adjoining surfaces ***1974, 1976.*** The angle ***θ'*, *θ"*** between the surfaces ***1972-1974,*** and ***1972-1976*** varies according to the design of the implant.

### ii. Hip Joint

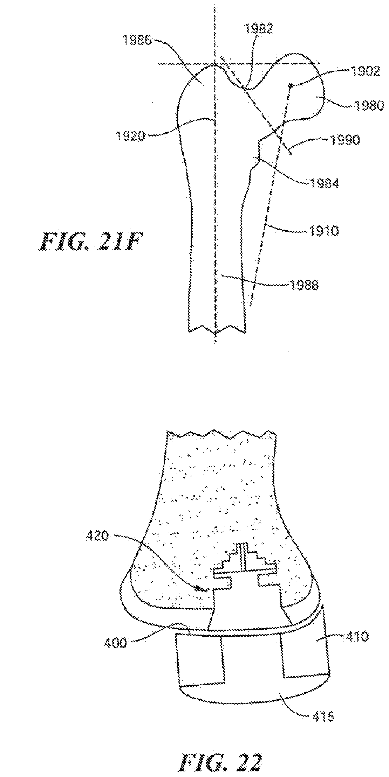

As illustrated in **FIG. 21F****,** the external geometry of the proximal femur includes the head ***1980,*** the neck ***1982,*** the lesser trochanter ***1984,*** the greater trochanter ***1986*** and the proximal femoral diaphysis. The relative positions of the trochanters ***1984, 1986,*** the femoral head center ***1902*** and the femoral shaft ***1988*** are correlated with the inclination of the neck-shaft angle. The mechanical axis ***1910*** and anatomic axis ***1920*** are also shown. Assessment of these relationships can change the reaming direction to achieve neutral alignment of the prosthesis with the femoral canal.

Using anteroposterior and lateral radiographs, measurements are made of the proximal and distal geometry to determine the size and optimal design of the implant.

Typically, after obtaining surgical access to the hip joint, the femoral neck ***1982*** is resected, e.g. along the line ***1990.*** Once the neck is resected, the medullary canal is reamed. Reaming can be accomplished, for example, with a conical or straight reamer, or a flexible reamer. The depth of reaming is dictated by the specific design of the implant. Once the canal has been reamed, the proximal reamer is prepared by serial rasping, with the rasp directed down into the canal.

### C. SURGICAL TOOLS

Further, surgical assistance can be provided by using a device applied to the outer surface of the articular cartilage or the bone, including the subchondral bone, in order to match the alignment of the articular repair system and the recipient site or the joint. The device can be round, circular, oval, ellipsoid, curved or irregular in shape. The shape can be selected or adjusted to match or enclose an area of diseased cartilage or an area slightly larger than the area of diseased cartilage or substantially larger than the diseased cartilage. The area can encompass the entire articular surface or the weight bearing surface. Such devices are typically preferred when replacement of a majority or an entire articular surface is contemplated.

Mechanical devices can be used for surgical assistance (*e.g.,* surgical tools), for example using gels, molds, plastics or metal. One or more electronic images or intraoperative measurements can be obtained providing object coordinates that define the articular and/or bone surface and shape. These objects' coordinates can be utilized to either shape the device, *e.g.* using a CAD/CAM technique, to be adapted to a patient's articular anatomy or, alternatively, to select a typically pre-made device that has a good fit with a patient's articular anatomy. The device can have a surface and shape that will match all or portions of the articular cartilage, subchondral bone and/or other bone surface and shape, *e.g.* similar to a "mirror image." The device can include, without limitation, one or more cut planes, apertures, slots and/or holes to accommodate surgical instruments such as drills, reamers, curettes, k-wires, screws and saws.

The device may have a single component or multiple components. The components may be attached to the unoperated and operated portions of the intra- or extra-articular anatomy. For example, one component may be attached to the femoral neck, while another component may be in contact with the greater or lesser trochanter. Typically, the different components can be used to assist with different parts of the surgical procedure. When multiple components are used, one or more components may also be attached to a different component rather than the articular cartilage, subchondral bone or other areas of osseous or non-osseous anatomy. For example, a tibial mold may be attached to a femoral mold and tibial cuts can be performed in reference to femoral cuts.

Components may also be designed to fit to the joint after an operative step has been performed. For example, in a knee, one component may be designed to fit all or portions of a distal femur before any cuts have been made, while another component may be designed to fit on a cut that has been made with the previously used mold or component. In a hip, one component may be used to perform an initial cut, for example through the femoral neck, while another subsequently used component may be designed to fit on the femoral neck after the cut, for example covering the area of the cut with a central opening for insertion of a reamer. Using this approach, subsequent surgical steps may also be performed with high accuracy, e.g. reaming of the marrow cavity.

In another embodiment, a guide may be attached to a mold to control the direction and orientation of surgical instruments. For example, after the femoral neck has been cut, a mold may be attached to the area of the cut, whereby it fits portions or all of the exposed bone surface. The mold may have an opening adapted for a reamer. Before the reamer is introduced a femoral reamer guide may be inserted into the mold and advanced into the marrow cavity. The position and orientation of the reamer guide may be determined by the femoral mold. The reamer can then be advanced over the reamer guide and the marrow cavity can be reamed with improved accuracy. Similar approaches are feasible in the knee and other joints.

All mold components may be disposable. Alternatively, some molds components may be re-usable. Typically, mold components applied after a surgical step such as a cut as been performed can be reusable, since a reproducible anatomic interface will have been established.

Interconnecting or bridging components may be used. For example, such interconnecting or bridging components may couple the mold attached to the joint with a standard, preferably unmodified or only minimally modified cut block used during knee or hip surgery. Interconnecting or bridging components may be made of plastic or metal. When made of metal or other hard material, they can help protect the joint from plastic debris, for example when a reamer or saw would otherwise get into contact with the mold.

The accuracy of the attachment between the component or mold and the cartilage or subchondral bone or other osseous structures is typically better than 2mm, more preferred better than 1mm, more preferred better than 0.7mm, more preferred better than 0.5mm, or even more preferred better than 0.5mm. The accuracy of the attachment between different components or between one or more molds and one or more surgical instruments is typically better than 2mm, more preferred better than 1mm, more preferred better than 0.7mm, more preferred better than 0.5mm, or even more preferred better than 0.5mm.

The angular error of any attachments or between any components or between components, molds, instruments and/or the anatomic or biomechanical axes is preferably less than 2 degrees, more preferably less than 1.5 degrees, more preferably less than 1 degree, and even more preferably less than 0.5 degrees. The total angular error is preferably less than 2 degrees, more preferably less than 1.5 degrees, more preferably less than 1 degree, and even more preferably less than 0.5 degrees.

Typically, a position will be chosen that will result in an anatomically desirable cut plane, drill hole, or general instrument orientation for subsequent placement of an articular repair system or for facilitating placement of the articular repair system. Moreover, the device can be designed so that the depth of the drill, reamer or other surgical instrument can be controlled, e.g., the drill cannot go any deeper into the tissue than defined by the device, and the size of the hole in the block can be designed to essentially match the size of the implant. Information about other joints or axis and alignment information of a joint or extremity can be included when selecting the position of these slots or holes. Alternatively, the openings in the device can be made larger than needed to accommodate these instruments. The device can also be configured to conform to the articular shape. The apertures, or openings, provided can be wide enough to allow for varying the position or angle of the surgical instrument, *e.g.,* reamers, saws, drills, curettes and other surgical instruments. An instrument guide, typically comprised of a relatively hard material, can then be applied to the device. The device helps orient the instrument guide relative to the three-dimensional anatomy of the joint.

The mold may contact the entire articular surface. In various embodiments, the mold can be in contact with only a portion of the articular surface. Thus, the mold can be in contact, without limitation, with: 100% of the articular surface; 80% of the articular surface; 50% of the articular surface; 30% of the articular surface; 30% of the articular surface; 20% of the articular surface; or10% or less of the articular surface. An advantage of a smaller surface contact area is a reduction in size of the mold thereby enabling cost efficient manufacturing and, more important, minimally invasive surgical techniques. The size of the mold and its surface contact areas have to be sufficient, however, to ensure accurate placement so that subsequent drilling and cutting can be performed with sufficient accuracy.

In various embodiments, the maximum diameter of the mold is less than 10cm. In other embodiments, the maximum diameter of the mold may be less than: 8cm; 5cm; 4cm; 3cm; or even less than 2cm.

The mold may be in contact with three or more surface points rather than an entire surface. These surface points may be on the articular surface or external to the articular surface. By using contact points rather than an entire surface or portions of the surface, the size of the mold may be reduced.

Reductions in the size of the mold can be used to enable minimally invasive surgery (MIS) in the hip, the knee, the shoulder and other joints. MIS technique with small molds will help to reduce intraoperative blood loss, preserve tissue including possibly bone, enable muscle sparing techniques and reduce postoperative pain and enable faster recovery. Thus, in one embodiment, the mold is used in conjunction with a muscle sparing technique. In another embodiment, the mold may be used with a bone sparing technique. In another embodiment, the mold is shaped to enable MIS technique with an incision size of less than 15cm, or, more preferred, less than 13cm, or, more preferred, less than 10cm, or, more preferred, less than 8cm, or, more preferred, less than 6cm.

The mold may be placed in contact with points or surfaces outside of the articular surface. For example, the mold can rest on bone in the intercondylar notch or the anterior or other aspects of the tibia or the acetabular rim or the lesser or greater trochanter. Optionally, the mold may only rest on points or surfaces that are external to the articular surface. Furthermore, the mold may rest on points or surfaces within the weight-bearing surface, or on points or surfaces external to the weight-bearing surface.

The mold may be designed to rest on bone or cartilage outside the area to be worked on, e.g. cut, drilled etc. In this manner, multiple surgical steps can be performed using the same mold. For example, in the knee, the mold may be stabilized against portions of the intercondylar notch, which can be selected external to areas to be removed for total knee arthroplasty or other procedures. In the hip, the mold may be attached external to the acetabular fossa, providing a reproducible reference that is maintained during a procedure, for example total hip arthroplasty. The mold may be affixed to the underlying bone, for example with pins or drills etc.

In additional embodiments, the mold may rest on the articular cartilage. The mold may rest on the subchondral bone or on structures external to the articular surface that are within the joint space or on structures external to the joint space. If the mold is designed to rest on the cartilage, an imaging test demonstrating the articular cartilage can be used in one embodiment. This can, for example, include ultrasound, spiral CT arthrography, MRI using, for example, cartilage displaying pulse sequences, or MRI arthrography. In another embodiment, an imaging test demonstrating the subchondral bone, e.g. CT or spiral CT, can be used and a standard cartilage thickness can be added to the scan. The standard cartilage thickness can be derived, for example, using an anatomic reference database, age, gender, and race matching, age adjustments and any method known in the art or developed in the future for deriving estimates of cartilage thickness. The standard cartilage thickness may, in some embodiments, be uniform across one or more articular surfaces or it can change across the articular surface.

The mold may be adapted to rest substantially on subchondral bone. In this case, residual cartilage can create some offset and inaccurate result with resultant inaccuracy in surgical cuts, drilling and the like. In one embodiment, the residual cartilage is removed in a first step in areas where the mold is designed to contact the bone and the subchondral bone is exposed. In a second step, the mold is then placed on the subchondral bone.

With advanced osteoarthritis, significant articular deformity can result. The articular surface(s) can become flattened. There can be cyst formation or osteophyte formation. "Tram track" like structures can form on the articular surface. In one embodiment, osteophytes or other deformities may be removed by the computer software prior to generation of the mold. The software can automatically, semi-automatically or manually with input from the user simulate surgical removal of the osteophytes or other deformities, and predict the resulting shape of the joint and the associated surfaces. The mold can then be designed based on the predicted shape. Intraoperatively, these osteophytes or other deformities can then also optionally be removed prior to placing the mold and performing the procedure. Alternatively, the mold can be designed to avoid such deformities. For example, the mold may only be in contact with points on the articular surface or external to the articular surface that are not affected or involved by osteophytes. The mold can rest on the articular surface or external to the articular surface on three or more points or small surfaces with the body of the mold elevated or detached from the articular surface so that the accuracy of its position cannot be affected by osteophytes or other articular deformities. The mold can rest on one or more tibial spines or portions of the tibial spines. Alternatively, all or portions of the mold may be designed to rest on osteophytes or other excrescences or pathological changes.

The surgeon can, optionally, make fine adjustments between the alignment device and the instrument guide. In this manner, an optimal compromise can be found, for example, between biomechanical alignment and joint laxity or biomechanical alignment and joint function, *e.g.* in a knee joint flexion gap and extension gap. By oversizing the openings in the alignment guide, the surgeon can utilize the instruments and insert them in the instrument guide without damaging the alignment guide. Thus, in particular if the alignment guide is made of plastic, debris will not be introduced into the joint. The position and orientation between the alignment guide and the instrument guide can be also be optimized with the use of, for example, interposed spacers, wedges, screws and other mechanical or electrical methods known in the art.

A surgeon may desire to influence joint laxity as well as joint alignment. This can be optimized for different flexion and extension, abduction, or adduction, internal and external rotation angles. For this purpose, for example, spacers can be introduced that are attached or that are in contact with one or more molds. The surgeon can intraoperatively evaluate the laxity or tightness of a joint using spacers with different thickness or one or more spacers with the same thickness. For example, spacers can be applied in a knee joint in the presence of one or more molds and the flexion gap can be evaluated with the knee joint in flexion. The knee joint can then be extended and the extension gap can be evaluated. Ultimately, the surgeon will select an optimal combination of spacers for a given joint and mold. A surgical cut guide can be applied to the mold with the spacers optionally interposed between the mold and the cut guide. In this manner, the exact position of the surgical cuts can be influenced and can be adjusted to achieve an optimal result. Thus, the position of a mold can be optimized relative to the joint, bone or cartilage for soft-tissue tension, ligament balancing or for flexion, extension, rotation, abduction, adduction, anteversion, retroversion and other joint or bone positions and motion. The position of a cut block or other surgical instrument may be optimized relative to the mold for soft-tissue tension or for ligament balancing or for flexion, extension, rotation, abduction, adduction, anteversion, retroversion and other joint or bone positions and motion. Both the position of the mold and the position of other components including cut blocks and surgical instruments may be optimized for soft-tissue tension or for ligament balancing or for flexion, extension, rotation, abduction, adduction, anteversion, retroversion and other joint or bone positions and motion.

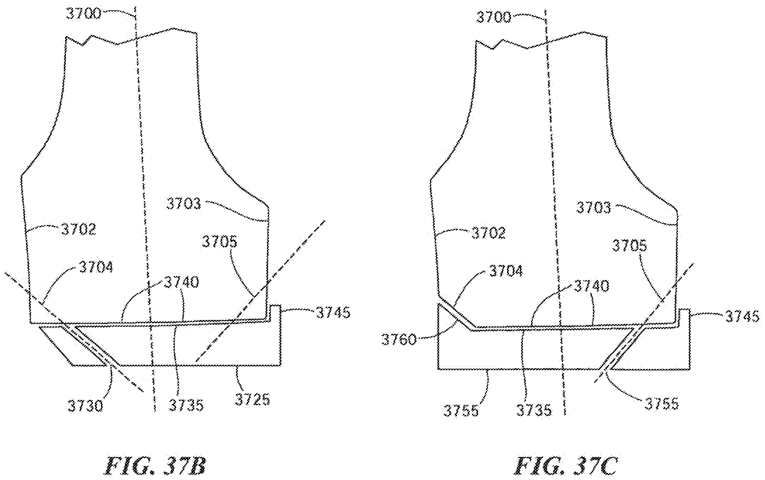

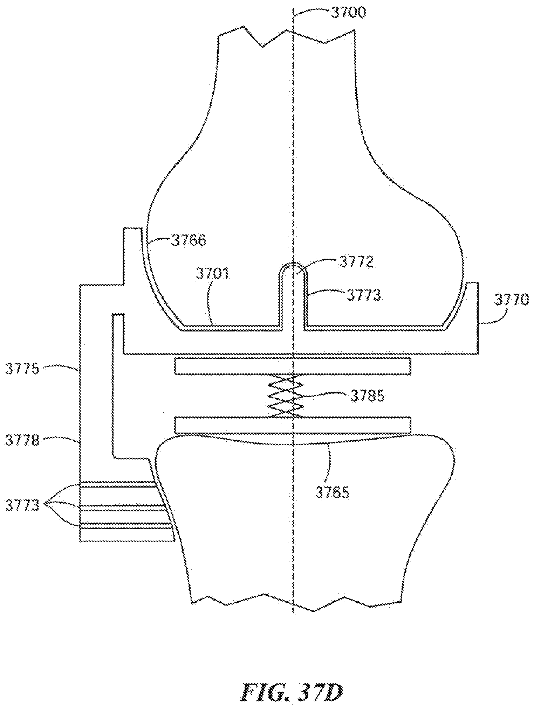

Someone skilled in the art will recognize other means for optimizing the position of the surgical cuts or other interventions. As stated above, expandable or ratchet-like devices may be utilized that can be inserted into the joint or that can be attached or that can touch the mold (see also **FIG. 37D**). Such devices can extend from a cutting block or other devices attached to the mold, optimizing the position of drill holes or cuts for different joint positions or they can be integrated inside the mold. Integration in the cutting block or other devices attached to the mold is preferable, since the expandable or ratchet-like mechanisms can be sterilized and re-used during other surgeries, for example in other patients. Optionally, the expandable or ratchet-like devices may be disposable. The expandable or ratchet like devices may extend to the joint without engaging or contacting the mold; alternatively, these devices may engage or contact the mold. Hinge-like mechanisms are applicable. Similarly, jack-like mechanisms are useful. In principal, any mechanical or electrical device useful for fine-tuning the position of the cut guide relative to the molds may be used. These embodiments are helpful for soft-tissue tension optimization and ligament balancing in different joints for different static positions and during joint motion.

A surgeon may desire to influence joint laxity as well as joint alignment. This can be optimized for different flexion and extension, abduction, or adduction, internal and external rotation angles. For this purpose, for example, spacers or expandable or ratchet-like can be utilized that can be attached or that can be in contact with one or more molds. The surgeon can intraoperatively evaluate the laxity or tightness of a joint using spacers with different thickness or one or more spacers with the same thickness or using such expandable or ratchet like devices. For example, spacers or a ratchet like device can be applied in a knee joint in the presence of one or more molds and the flexion gap can be evaluated with the knee joint in flexion. The knee joint can then be extended and the extension gap can be evaluated. Ultimately, the surgeon will select an optimal combination of spacers or an optimal position for an expandable or ratchet-like device for a given joint and mold. A surgical cut guide can be applied to the mold with the spacers or the expandable or ratchet-like device optionally interposed between the mold and the cut guide or, in select embodiments, between the mold and the joint or the mold and an opposite articular surface. In this manner, the exact position of the surgical cuts can be influenced and can be adjusted to achieve an optimal result. Someone skilled in the art will recognize other means for optimizing the position of the surgical cuts or drill holes. For example, expandable or ratchet-like devices can be utilized that can be inserted into the joint or that can be attached or that can touch the mold. Hinge-like mechanisms are applicable. Similarly, jack-like mechanisms are useful. In principal, any mechanical or electrical device useful for fine-tuning the position of the cut guide relative to the molds can be used.

The template and any related instrumentation such as spacers or ratchets can be combined with a tensiometer to provide a better intraoperative assessment of the joint. The tensiometer can be utilized to further optimize the anatomic alignment and tightness of the joint and to improve post-operative function and outcomes. Optionally, local contact pressures may be evaluated intraoperatively, for example using a sensor like the ones manufactured by Tekscan, South Boston, Mass. The contact pressures can be measured between the mold and the joint or between the mold and any attached devices such as a surgical cut block.

The template may be a mold that can be made of a plastic or polymer. The mold may be produced by rapid prototyping technology, in which successive layers of plastic are laid down, as know in the art. In other embodiments, the template or portions of the template can be made of metal. The mold can be milled or made using laser based manufacturing techniques.

The template may be casted using rapid prototyping and, for example, lost wax technique. It may also be milled. For example, a preformed mold with a generic shape can be used at the outset, which can then be milled to the patient specific dimensions. The milling may only occur on one surface of the mold, preferably the surface that faces the articular surface. Milling and rapid prototyping techniques may be combined.

Curable materials may be used which can be poured into forms that are, for example, generated using rapid prototyping. For example, liquid metal may be used. Cured materials may optionally be milled or the surface can be further refined using other techniques.

Metal inserts may be applied to plastic components. For example, a plastic mold may have at least one guide aperture to accept a reaming device or a saw. A metal insert may be used to provide a hard wall to accept the reamer or saw. Using this or similar designs can be useful to avoid the accumulation of plastic or other debris in the joint when the saw or other surgical instruments may get in contact with the mold. Other hard materials can be used to serve as inserts. These can also include, for example, hard plastics or ceramics.

In another embodiment, the mold does not have metallic inserts to accept a reaming device or saw. The metal inserts or guides may be part of an attached device, which is typically in contact with the mold. A metallic drill guide or a metallic saw guide may thus, for example, have metallic or hard extenders that reach through the mold thereby, for example, also stabilizing any devices applied to the mold against the physical body of the mold.

The template may not only be used for assisting the surgical technique and guiding the placement and direction of surgical instruments. In addition, the templates can be utilized for guiding the placement of the implant or implant components. For example, in the hip joint, tilting of the acetabular component is a frequent problem with total hip arthroplasty. A template can be applied to the acetabular wall with an opening in the center large enough to accommodate the acetabular component that the surgeon intends to place. The template can have receptacles or notches that match the shape of small extensions that can be part of the implant or that can be applied to the implant. For example, the implant can have small members or extensions applied to the twelve o'clock and six o'clock positions. By aligning these members with notches or receptacles in the mold, the surgeon can ensure that the implant is inserted without tilting or rotation. These notches or receptacles can also be helpful to hold the implant in place while bone cement is hardening in cemented designs.

One or more templates can be used during the surgery. For example, in the hip, a template can be initially applied to the proximal femur that closely approximates the 3D anatomy prior to the resection of the femoral head. The template can include an opening to accommodate a saw. The opening is positioned to achieve an optimally placed surgical cut for subsequent reaming and placement of the prosthesis. A second template can then be applied to the proximal femur after the surgical cut has been made. The second template can be useful for guiding the direction of a reamer prior to placement of the prosthesis. As can be seen in this, as well as in other examples, templates can be made for joints prior to any surgical intervention. However, it is also possible to make templates that are designed to fit to a bone or portions of a joint after the surgeon has already performed selected surgical procedures, such as cutting, reaming, drilling, etc. The template can account for the shape of the bone or the joint resulting from these procedures.

In certain embodiments, the surgical assistance device comprises an array of adjustable, closely spaced pins (*e.g.,* plurality of individually moveable mechanical elements). One or more electronic images or intraoperative measurements can be obtained providing object coordinates that define the articular and/or bone surface and shape. These objects' coordinates can be entered or transferred into the device, for example manually or electronically, and the information can be used to create a surface and shape that will match all or portions of the articular and/or bone surface and shape by moving one or more of the elements, *e.g.* similar to an "image." The device can include slots and holes to accommodate surgical instruments such as drills, curettes, k-wires, screws and saws. The position of these slots and holes may be adjusted by moving one or more of the mechanical elements. Typically, a position will be chosen that will result in an anatomically desirable cut plane, reaming direction, or drill hole or instrument orientation for subsequent placement of an articular repair system or for facilitating the placement of an articular repair system.

Information about other joints or axis and alignment information of a joint or extremity can be included when selecting the position of the, without limitation, cut planes, apertures, slots or holes on the template, in accordance with one embodiment. The biomechanical and/or anatomic axes may be derived using above-described imaging techniques including, without limitation, a standard radiograph, including a load bearing radiograph, for example an upright knee x-ray or a whole leg length film (e.g., hip to foot) These radiographs may be acquired in different projections, for example anteroposterior, posteroanterior, lateral, oblique etc. The biomechanical and anatomic axes may also be derived using other imaging modalities such as CT scan or MRI scan, a CT scout scan or MRI localized scans through portions or all of the extremity, either alone or in combination, as described in above embodiments. For example, when total or partial knee arthroplasty is contemplated, a spiral CT scan may be obtained through the knee joint. The spiral CT scan through the knee joint serves as the basis for generating the negative contour template(s)/mold(s) that will be affixed to portions or all of the knee joint. Additional CT or MRI scans may be obtained through the hip and ankle joint. These may be used to define the centroids or centerpoints in each joint or other anatomic landmarks, for example, and then to derive the biomechanical and other axes.

In another embodiment, the biomechanical axis may be established using non-image based approaches including traditional surgical instruments and measurement tools such as intramedullary rods, alignment guides and also surgical navigation. For example, in a knee joint, optical or radiofrequency markers can be attached to the extremity. The lower limb may then be rotated around the hip joint and the position of the markers can be recorded for different limb positions. The center of the rotation will determine the center of the femoral head. Similar reference points may be determined in the ankle joint etc. The position of the templates or, more typically, the position of surgical instruments relative to the templates may then be optimized for a given biomechanical load pattern, for example in varus or valgus alignment. Thus, by performing these measurements pre- or intraoperatively, the position of the surgical instruments may be optimized relative to the molds and the cuts can be placed to correct underlying axis errors such as varus or valgus malalignment or ante- or retroversion.

Upon imaging, a physical template of a joint, such as a knee joint, or hip joint, or ankle joint or shoulder joint is generated, in accordance with one embodiment. The template can be used to perform image guided surgical procedures such as partial or complete joint replacement, articular resurfacing, or ligament repair. The template may include reference points or opening or apertures for surgical instruments such as drills, saws, burrs and the like.

In order to derive the preferred orientation of drill holes, cut planes, saw planes and the like, openings or receptacles in said template or attachments will be adjusted to account for at least one axis. The axis can be anatomic or biomechanical, for example, for a knee joint, a hip joint, an ankle joint, a shoulder joint or an elbow joint.

In one embodiment, only a single axis is used for placing and optimizing such drill holes, saw planes, cut planes, and or other surgical interventions. This axis may be, for example, an anatomical or biomechanical axis. In a preferred embodiment, a combination of axis and/or planes can be used for optimizing the placement of the drill holes, saw planes, cut planes or other surgical interventions. For example, two axes (e.g., one anatomical and one biomechanical) can be factored into the position, shape or orientation of the 3D guided template and related attachments or linkages. For example, two axes, (e.g., one anatomical and biomechanical) and one plane (e.g., the top plane defined by the tibial plateau), can be used. Alternatively, two or more planes can be used (e.g., a coronal and a sagittal plane), as defined by the image or by the patients anatomy.

Angle and distance measurements and surface topography measurements may be performed in these one or more, preferably two or more, preferably three or more multiple planes, as necessary. These angle measurements can, for example, yield information on varus or valgus deformity, flexion or extension deficit, hyper or hypo--flexion or hyper- or hypo-extension, abduction, adduction, internal or external rotation deficit, or hyper-or hypo-abduction, hyper- or hypo-adduction, hyper- or hypo- internal or external rotation.

Single or multi-axis line or plane measurements can then be utilized to determine preferred angles of correction, e.g., by adjusting surgical cut or saw planes or other surgical interventions. Typically, two axis corrections will be preferred over a single axis correction, a two plane correction will be preferred over a single plane correction and so forth.

In accordance with another embodiment, more than one drilling, cut, boring and/or reaming or other surgical intervention is performed for a particular treatment such as the placement of a joint resurfacing or replacing implant, or components thereof. These two or more surgical interventions (e.g., drilling, cutting, reaming, sawing) are made in relationship to a biomechanical axis, and/or an anatomical axis and/or an implant axis. The 3D guidance template or attachments or linkages thereto include two or more openings, guides, apertures or reference planes to make at least two or more drillings, reamings, borings, sawings or cuts in relationship to a biomechanical axis, an anatomical axis, an implant axis or other axis derived therefrom or related thereto.

While in simple embodiments it is possible that only a single cut or drilling will be made in relationship to a biomechanical axis, an anatomical axis, an implant axis and/or an axis related thereto, in most meaningful implementations, two or more drillings, borings, reamings, cutting and/or sawings will be performed or combinations thereof in relationship to a biomechanical, anatomical and/or implant axis.

For example, an initial cut may be placed in relationship to a biomechanical axis of particular joint. A subsequent drilling, cut or other intervention can be performed in relation to an anatomical axis. Both can be designed to achieve a correction in a biomechanical axis and/or anatomical axis. In another example, an initial cut can be performed in relationship to a biomechanical axis, while a subsequent cut is performed in relationship to an implant axis or an implant plane. Any combination in surgical interventions and in relating them to any combination of biomechanical, anatomical, implant axis or planes related thereto is possible. In many embodiments, it is desirable that a single cut or drilling be made in relationship to a biomechanical or anatomical axis. Subsequent cuts or drillings or other surgical interventions can then be made in reference to said first intervention. These subsequent interventions can be performed directly off the same 3D guidance template or they can be performed by attaching surgical instruments or linkages or reference frames or secondary or other templates to the first template or the cut plane or hole and the like created with the first template.

**FIG. 22** shows an example of a surgical tool **410** having one surface **400** matching the geometry of an articular surface of the joint. Also shown is an aperture **415** in the tool **410** capable of controlling drill depth and width of the hole and allowing implantation or insertion of implant **420** having a press-fit design.

In another embodiment, a frame can be applied to the bone or the cartilage in areas other than the diseased bone or cartilage. The frame can include holders and guides for surgical instruments. The frame can be attached to one or preferably more previously defined anatomic reference points. Alternatively, the position of the frame can be cross-registered relative to one, or more, anatomic landmarks, using an imaging test or intraoperative measurement, for example one or more fluoroscopic images acquired intraoperatively. One or more electronic images or intraoperative measurements including using mechanical devices can be obtained providing object coordinates that define the articular and/or bone surface and shape. These objects' coordinates can be entered or transferred into the device, for example manually or electronically, and the information can be used to move one or more of the holders or guides for surgical instruments. Typically, a position will be chosen that will result in a surgically or anatomically desirable cut plane or drill hole orientation for subsequent placement of an articular repair system. Information about other joints or axis and alignment information of a joint or extremity can be included when selecting the position of these slots or holes.

Furthermore, re-useable tools (*e.g.,* molds) can be also be created and employed. Non-limiting examples of re-useable materials include putties and other deformable materials (*e.g.,* an array of adjustable closely spaced pins that can be configured to match the topography of a joint surface). In other embodiments, the molds may be made using balloons. The balloons can optionally be filled with a hardening material. A surface can be created or can be incorporated in the balloon that allows for placement of a surgical cut guide, reaming guide, drill guide or placement of other surgical tools. The balloon or other deformable material can be shaped intraoperatively to conform to at least one articular surface. Other surfaces can be shaped in order to be parallel or perpendicular to anatomic or biomechanical axes. The anatomic or biomechanical axes can be found using an intraoperative imaging test or surgical tools commonly used for this purpose in hip, knee or other arthroplasties.

In various embodiments, the template may include a reference element, such as a pin, that upon positioning of the template on the articular surface, establishes a reference plane relative to a biomechanical axis or an anatomical axis or plane of a limb. For example, in a knee surgery the reference element may establish a reference plane from the center of the hip to the center of the ankle. In other embodiments, the reference element may establish an axis that subsequently be used a surgical tool to correct an axis deformity.

In these embodiments, the template can be created directly from the joint during surgery or, alternatively, created from an image of the joint, for example, using one or more computer programs to determine object coordinates defining the surface contour of the joint and transferring (*e.g.,* dialing-in) these co-ordinates to the tool. Subsequently, the tool can be aligned accurately over the joint and, accordingly, the surgical instrument guide or the implant will be more accurately placed in or over the articular surface.

In both single-use and re-useable embodiments, the tool can be designed so that the instrument controls the depth and/or direction of the drill, *i.e.,* the drill cannot go any deeper into the tissue than the instrument allows, and the size of the hole or aperture in the instrument can be designed to essentially match the size of the implant. The tool can be used for general prosthesis implantation, including, but not limited to, the articular repair implants described herein and for reaming the marrow in the case of a total arthroplasty.

These surgical tools (devices) can also be used to remove an area of diseased cartilage and underlying bone or an area slightly larger than the diseased cartilage and underlying bone. In addition, the device can be used on a "donor," *e.g.,* a cadaveric specimen, to obtain implantable repair material. The device is typically positioned in the same general anatomic area in which the tissue was removed in the recipient. The shape of the device is then used to identify a donor site providing a seamless or near seamless match between the donor tissue sample and the recipient site. This can be achieved by identifying the position of the device in which the articular surface in the donor, *e.g.* a cadaveric specimen, has a seamless or near seamless contact with the inner surface when applied to the cartilage.

The device can be molded, rapid prototyped, machine and/or formed based on the size of the area of diseased cartilage and based on the curvature of the cartilage or the underlying subchondral bone or a combination of both or using adjacent structures inside or external to the joint space. The device can take into consideration surgical removal of, for example, the meniscus, in arriving at a joint surface configuration.

In one embodiment, the device can then be applied to the donor, (e.g., a cadaveric specimen) and the donor tissue can be obtained with use of a blade or saw or other tissue removing device. The device can then be applied to the recipient in the area of the joint and the diseased cartilage, where applicable, and underlying bone can be removed with use of a blade or saw or other tissue cutting device whereby the size and shape of the removed tissue containing the diseased cartilage will closely resemble the size and shape of the donor tissue. The donor tissue can then be attached to the recipient site. For example, said attachment can be achieved with use of screws or pins (e.g., metallic, non-metallic or bioresorable) or other fixation means including but not limited to a tissue adhesive. Attachment can be through the cartilage surface or alternatively, through the marrow space.

The implant site can be prepared with use of a robotic device. The robotic device can use information from an electronic image for preparing the recipient site.

Identification and preparation of the implant site and insertion of the implant can be supported by a surgical navigation system. In such a system, the position or orientation of a surgical instrument with respect to the patient's anatomy can be tracked in real-time in one or more 2D or 3D images. These 2D or 3D images can be calculated from images that were acquired preoperatively, such as MR or CT images. Non-image based surgical navigation systems that find axes or anatomical structures, for example with use of joint motion, can also be used. The position and orientation of the surgical instrument as well as the mold including alignment guides, surgical instrument guides, reaming guides, drill guides, saw guides, etc. can be determined from markers attached to these devices. These markers can be located by a detector using, for example, optical, acoustical or electromagnetic signals.

Identification and preparation of the implant site and insertion of the implant can also be supported with use of a C-arm system. The C-arm system can afford imaging of the joint in one or, preferably, multiple planes. The multiplanar imaging capability can aid in defining the shape of an articular surface. This information can be used to select an implant with a good fit to the articular surface. Currently available C-arm systems also afford cross-sectional imaging capability, for example for identification and preparation of the implant site and insertion of the implant. C-arm imaging can be combined with administration of radiographic contrast.

In various embodiments, the surgical devices described herein can include one or more materials that harden to form a mold of the articular surface. In preferred embodiments, the materials used are biocompatible, such as, without limitation, acylonitrile butadiene styrene, polyphenylsulfone and polycarbonate. As used herein "biocompatible" shall mean any material that is not toxic to the body (e.g., produces a negative reaction under ISO 10993 standards, incorporated herein by reference). In various embodiments, these biocompatible materials may be compatible with rapid prototyping techniques.

In further embodiments, the mold material is capable of heat sterilization without deformation. An exemplary mold material is polyphenylsulfone, which does not deform up to a temperature of 207 degrees Celsius. Alternatively, the mold may be capable of sterilization using gases, e.g. ethyleneoxide. The mold may be capable of sterilization using radiation, e.g. γ-radiation. The mold may be capable of sterilization using hydrogen peroxide or other chemical means. The mold may be capable of sterilization using any one or more methods of sterilization known in the art or developed in the future.

A wide-variety of materials capable of hardening in situ include polymers that can be triggered to undergo a phase change, for example polymers that are liquid or semi-liquid and harden to solids or gels upon exposure to air, application of ultraviolet light, visible light, exposure to blood, water or other ionic changes. (See, also, U.S. Pat. No. 6,443,988 to Felt et al. issued Sep. 3, 2002 and documents cited therein). Non-limiting examples of suitable curable and hardening materials include polyurethane materials (e.g., U.S. Pat. No. 6,443,988 to Felt et al., U.S. Pat. No. 5,288,797 to Khalil issued Feb. 22, 1994, U.S. Pat. No. 4,098,626 to Graham et al. issued Jul. 4, 1978 and U.S. Pat. No. 4,594,380 to Chapin et al. issued Jun. 10, 1986; and Lu et al. (2000) BioMaterials 21(15): 1595-1605 describing porous poly(L-lactide acid foams); hydrophilic polymers as disclosed, for example, in U.S. Pat. No. 5,162,430; hydrogel materials such as those described in Wake et al. (1995) Cell Transplantation 4(3):275-279, Wiese et al. (2001) J. Biomedical Materials Research 54(2):179-188 and Marler et al. (2000) Plastic Reconstruct. Surgery 105(6):2049-2058; hyaluronic acid materials (e.g., Duranti et al. (1998) Dermatologic Surgery 24(12):1317-1325); expanding beads such as chitin beads (e.g., Yusof et al. (2001) J. Biomedical Materials Research 54(1):59-68); crystal free metals such as Liquidmetals.RTM., and/or materials used in dental applications (See, e.g., Brauer and Antonucci, "Dental Applications" pp. 257-258 in "Concise Encyclopedia of Polymer Science and Engineering" and U.S. Pat. No. 4,368,040 to Weissman issued January 11, 1983). Any biocompatible material that is sufficiently flowable to permit it to be delivered to the joint and there undergo complete cure in situ under physiologically acceptable conditions can be used. The material can also be biodegradable.

The curable materials can be used in conjunction with a surgical tool as described herein. For example, the surgical tool can be a template that includes one or more apertures therein adapted to receive injections and the curable materials can be injected through the apertures. Prior to solidifying in situ the materials will conform to the articular surface (subchondral bone and/or articular cartilage) facing the surgical tool and, accordingly, will form a mirror image impression of the surface upon hardening, thereby recreating a normal or near normal articular surface.

In addition, curable materials or surgical tools can also be used in conjunction with any of the imaging tests and analysis described herein, for example by molding these materials or surgical tools based on an image of a joint. For example, rapid prototyping may be used to perform automated construction of the template. The rapid prototyping may include the use of, without limitation, 3D printers, stereolithography machines or selective laser sintering systems. Rapid prototyping is a typically based on computer-aided manufacturing (CAM). Although rapid prototyping traditionally has been used to produce prototypes, they are now increasingly being employed to produce tools or even to manufacture production quality parts. In an exemplary rapid prototyping method, a machine reads in data from a CAD drawing, and lays down successive millimeter-thick layers of plastic or other engineering material, and in this way the template can be built from a long series of cross sections. These layers are glued together or fused (often using a laser) to create the cross section described in the CAD drawing.

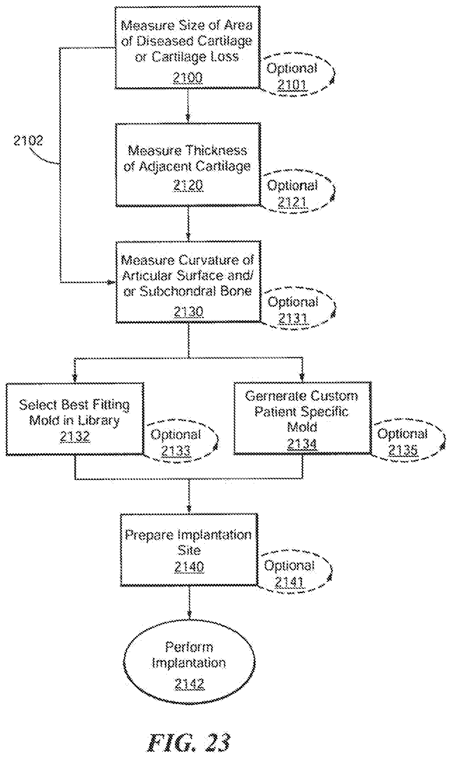

**FIG. 23** is a flow chart illustrating the steps involved in designing a mold for use in preparing a joint surface. Optionally, the first step can be to measure the size of the area of the diseased cartilage or cartilage loss ***2100.*** Once the size of the cartilage loss has been measured, the user can measure the thickness of the adjacent cartilage ***2120,*** prior to measuring the curvature of the articular surface and/or the subchondral bone ***2130.*** Alternatively, the user can skip the step of measuring the thickness of the adjacent
cartilage ***2102.*** Once an understanding and determination of the shape of the subchondral bone is determined, either a mold can be selected from a library of molds ***3132*** or a patient specific mold can be generated ***2134.*** In either event, the implantation site is then prepared ***2140*** and implantation is performed ***2142.*** Any of these steps can be repeated by the optional repeat steps ***2101, 2121, 2131, 2133, 2135, 2141.***

A variety of techniques can be used to derive the shape of the template, as described above. For example, a few selected CT slices through the hip joint, along with a full spiral CT through the knee joint and a few selected slices through the ankle joint can be used to help define the axes if surgery is contemplated of the knee joint. Once the axes are defined, the shape of the subchondral bone can be derived, followed by applying standardized cartilage loss.

Methodologies for stabilizing the 3D guidance templates will now be described. The 3D guide template may be stabilized using multiple surgical tools such as, without limitation: K-wires; a drill bit anchored into the bone and left within the template to stabilize it against the bone; one or more convexities or cavities on the surface facing the cartilage; bone stabilization against intra/extra articular surfaces, optionally with extenders, for example, from an articular surface onto an extra-articular surface; and/or stabilization against newly placed cuts or other surgical interventions.

Specific anatomic landmarks may be selected in the design and make of the 3D guide template in order to further optimize the anatomic stabilization. For example, a 3D guidance template may be designed to cover portions or all off an osteophyte or bone spur in order to enhance anchoring of the 3D guide template against the underlying articular anatomy. The 3D guidance template may be designed to the shape of a trochlear or intercondylar notch and can encompass multiple anatomic areas such as a trochlea, a medial and a lateral femoral condyle at the same time. In the tibia, a 3D guide template may be designed to encompass a medial and lateral tibial plateau at the same time and it can optionally include the tibial spine for optimized stabilization and cross-referencing. In a hip, the fovea capitis may be utilized in order to stabilize a 3D guide template. Optionally, the surgeon may elect to resect the ligamentum capitis femoris in order to improve the stabilization. Also in the hip, an acetabular mold can be designed to extend into the region of the tri-radiate cartilage, the medial, lateral, superior, inferior, anterior and posterior acetabular wall or ring. By having these extensions and additional features for stabilization, a more reproducible position of the 3D template can be achieved with resulted improvement in accuracy of the surgical procedure. Typically, a template with more than one convexity or concavity or multiple convexities or concavities will provide better cross-referencing in the anatomic surface and higher accuracy and higher stabilization than compared to a mold that has only few surface features such as a singular convexity. Thus, in order to improve the implementation and intraoperative accuracy, careful surgical planning and preoperative planning is desired, that encompasses preferably more than one convexity, more preferred more than two convexities and even more preferred more than three convexities, or that encompasses more than one concavity, more preferred more than two concavities or even more preferred more than three concavities on an articular surface or adjoined surface, including bone and cartilage outside the weight-bearing surface.

In an even more preferred embodiment, more than one convexity and concavity, more preferred more than two convexities and concavities and even more preferred more then three convexities and concavities are included in the surface of the mold in order to optimize the intraoperative cross-referencing and in order to stabilize the mold prior to any surgical intervention.

Turning now to particular 3D surgical template configurations and to templates for specific joint applications which are intended to teach the concept of the design as it would then apply to other joints in the body:

### i. 3D Guidance Template Configurations/Positioning

The 3D guidance template may include a surface that duplicates the inner surface of an implant or an implant component, and/or that conforms to an articular surface, at least partially, in accordance with an embodiment. More than one of the surfaces of the template may match or conform to one or more of the surfaces or portions of one or more of these surfaces of an implant, implant component, and/or articular surface.

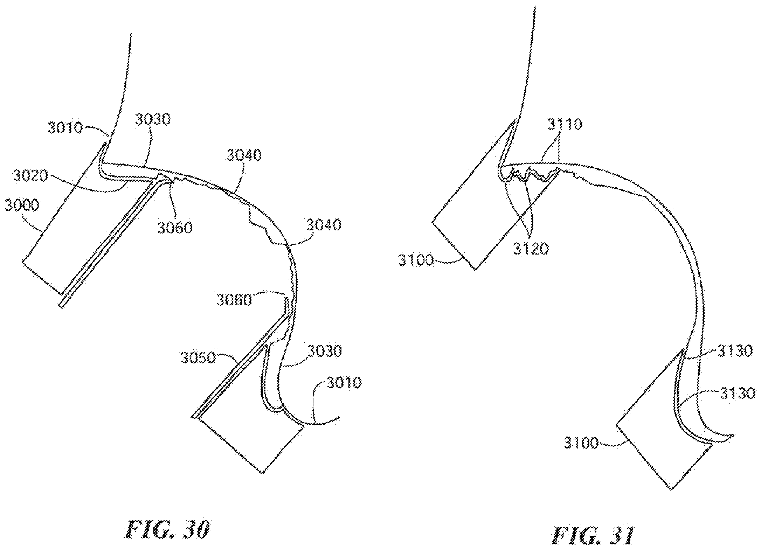

**FIG. 30** shows an example of a 3D guidance template **3000** in a hip joint, in accordance with one embodiment, wherein the template has extenders **3010** extending beyond the margin of the joint to provide for additional stability and to fix the template in place. The surface of the template facing the joint **3020** is a mirror image of a portion of the joint that is not affected by the arthritic process **3030.** By designing the template to be a mirror image of at least a portion of the joint that is not affected by the arthritic process, greater reproducibility in placing the template can be achieved. In this design, the template spares the arthritic portions **3040** of the joint and does not include them in its joint facing surface. The template can optionally have metal sleeves **3050** to accommodate a reamer or other surgical instruments, to protect a plastic. The metal sleeves or, optionally, the template can also include stops **3060** to limit the advancement of a surgical instrument once a predefined depth has been reached.

**FIG. 31** shows another embodiment of a 3D guidance template **3100** for an acetabulum, in accordance with one embodiment. The articular surface is roughened **3110** in some sections by the arthritic process. At least a portion of the template **3120** is made to be a mirror image of the articular surface altered by the arthritic process **3110.** By matching the template to the joint in areas where it is altered by the arthritic process improved intraoperative localization and improved fixation can be achieved. In other section, the template can be matched to portions of the joint that are not altered by the arthritic process **3130.**

**FIGS. 36A-D** show a knee joint with a femoral condyle **3600** including a normal **3610** and arthritic **3620** region, in accordance with various embodiments. The interface **3630** between normal **3610** and arthritic **3620** tissue is shown. The template is designed to guide a posterior cut **3640** using a guide plane **3650** or guide aperture **3660.**

In one embodiment shown in **FIG. 36A** the surface of the template facing the joint **3670** is a mirror image of at least portions of the surface of the joint that is healthy or substantially unaffected by the arthritic process. A recessed area **3670** can be present to avoid contact with the diseased joint region. This design can be favorable when an imaging test is used that does not provide sufficient detail about the diseased region of the joint to accurately generate a template.

In a similar embodiment shown in **FIG. 36B** the surface of the template facing the joint **3670** is a mirror image of at least portions of the surface of the joint that is healthy or substantially unaffected by the arthritic process. The diseased area **3620** is covered by the template, but the template is not substantially in contact with it.

In another embodiment shown in **FIG. 36C** the surface of the template facing the joint **3670** is a mirror image of at least portions of the surface of the joint that are arthritic. The diseased area **3620** is covered by the template, and the template is in close contact with it. This design can be advantageous to obtain greater accuracy in positioning the template if the arthritic area is well defined on the imaging test, e.g. with high resolution spiral CT or near isotropic MRI acquisitions or MRI with image fusion. This design can also provide enhanced stability during surgical interventions by more firmly fixing the template against the irregular underlying surface.

In another embodiment shown in **FIG. 36D** the surface of the template facing the joint **3670** is a mirror image of at least portions of the surface of the joint that are arthritic. The diseased area **3620** is covered by the template, and the template is in close contact with it. Moreover, healthy or substantially normal regions **3610** are covered by the template and the template is in close contact with them. The template is also closely mirroring the shape of the interface between substantially normal or near normal and diseased joint tissue **3630.** This design can be advantageous to obtain even greater accuracy in positioning the template due to the change in surface profile or contour at the interface and resultant improved placement of the template on the joint surface. This design can also provide enhanced stability during surgical interventions by more firmly fixing and anchoring the template against the underlying surface and the interface **3630.**

The template may include guide apertures or reference points for two or more planes, or at least one of a cut plane and one of a drill hole or reaming opening for a peg or implant stem, in accordance with one embodiment.

The distance between two opposing, articulating implant components may be optimized intraoperatively for different pose angles of the joint or joint positions, such as different degrees of section, extension, abduction, adduction, internal and external rotation. For example, spacers, typically at least partially conforming to the template, may be placed between the template of the opposite surface, where the opposite surface can be the native, uncut joint, the cut joint, the surgically prepared joint, the trial implant, or the definitive implant component for that articular surface. Alternatively, spacers may be placed between the template and the articular surface for which it will enable subsequent surgical interventions. For example, by placing spacers between a tibial template and the tibia, the tibial cut height can be optimized. The thicker the spacer, or the more spacers interposed between the tibial template and the tibial plateau, the less deep the cut will be, *i.e.* the less bone will be removed from the top of the tibia.

The spacers may be non-conforming to the template, *e.g.* they may be of a flat nature. The spacers may be convex or concave or include multiple convexities or concavities. The spacers may be partially conforming to the template. For example, in one embodiment, the surface of the spacer optionally facing the articular surface can be molded and individualized to the articular surface, thereby forming a template/mold, while the opposite surface of the spacer can be flat or curved or have any other non-patient specific design. The opposite surface may allow for placement of blocks or other surgical instruments or for linkages to other surgical instruments and measurement devices.

In another embodiment, the template may include multiple slots spaced at equal distance or at variable distances wherein these slots allow to perform cuts at multiple cut heights or cut depths that can be decided on intraoperatively. In another embodiment, the template may include a ratchet-like mechanism wherein the ratchet can be placed between the articular surface and the template or between the template and the opposite surface wherein the opposite surface may include the native, uncut opposite surface, the cut opposite surface, an opposite surface template, a trial implant or the implant component designed for the opposite surface. By using a ratchet-like device, soft tissue tension can be optimized, for example, for different pose angles of the joint or joint positions such as flexion, extension, abduction, adduction, internal rotation and external rotation at one or more degrees for each direction.

Optimizing soft tissue tension will improve joint function that advantageously enhances postoperative performance. Soft tissue tension may, for example, be optimized with regard to ligament tension or muscle tension but also capsular tension. In the knee joint, soft tissue tension optimization includes typically ligament balancing, *e.g.* the cruciate ligaments and/or the collateral ligaments, for different degrees of knee flexion and knee extension.

In a preferred embodiment, a 3D guidance template may attach to two or more points on the joint. In an even more preferred embodiment, a template may attach to three or more points on the joint, even more preferred four or more points on the joint, even more preferred five or more points on the joint, even more preferred six or more points on the joint, even more preferred seven or more points on the joint, even more preferred ten or more points on the joint, even more preferred portions for the entire surface to be replaced.

In another embodiment, the template may include one or more linkages for surgical instruments. The linkages may also be utilized for attaching other measurement devices such as alignment guides, intramedullary guides, laser pointing devices, laser measurement devices, optical measurement devices, radio frequency measurement devices, surgical navigation and the like. Someone skilled in the art will recognize many surgical instruments and measurement in alignment devices may be attached to the template. Alternatively, these surgical instruments or alignment devices may be included within the template.

In another embodiment, a link or a linkage may be attached or may be incorporated or may be part of a template that rests on a first articular surface. Said link or linkage may further extend to a second articular surface which is typically an opposing articular surface. Said link or linkage can thus help cross-reference the first articular surface with the second articular surface, ultimately assisting the performance of surgical interventions on the second articular surface using the cross reference to the first articular surface. The second articular surface may optionally be cut with a second template. Alternatively, the second articular surface may be cut using a standard surgical instrument, non-individualized, that is cross referenced via the link to the surgical mold placed on the first articular surface. The link or linkage may include adjustment means, such as ratchets, telescoping devices and the like to optimize the spatial relationship between the first articular surface and the second, opposing articular surface. This optimization may be performed for different degrees of joint flexion, extension, abduction, adduction and rotation.

In another embodiment, the linkage may be made to the cut articular surface or, more general, an articular surface that has been altered using a template and related surgical intervention. Thus, cross reference can be made from the first articular surface from a mold attached to said first articular surface, the mold attached to a surgically altered, for example, cut articular surface, the surgical instrument attached to said articular surface altered using the mold, *e.g.* cut or drilled, and the like. Someone skilled in the art will easily recognize multiple different variations of this approach. Irrespective of the various variations, in a first step the articular surface is surgically altered, for example, via cutting, drilling or reaming using a mold while in the second step cross reference is established with a second articular surface.

By establishing cross reference between said first and said second articular surface either via the template and/or prior to or after a surgical intervention, the surgical intervention performed on the second articular surface can be performed using greater accuracy and improved usability in relation to said articulating, opposing first articular surface.

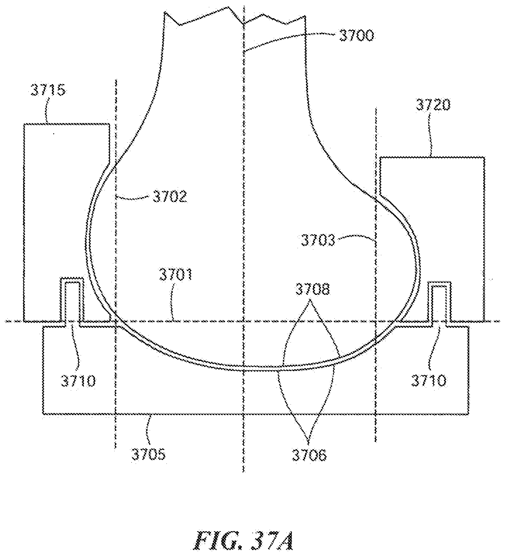

**FIGS. 37A-D** show multiple templates with linkages on the same articular surface (**A-C**) and to an opposing articular surface (**D**), in accordance with various embodiments. The biomechanical axis is denoted as **3700.** A horizontal femoral cut **3701,** an anterior femoral cut **3702,** a posterior femoral cut **3703,** an anterior chamfer cut **3704** and a posterior chamfer cut **3705** are planned in this example. A first template **3705** is applied in order to determine the horizontal cut plane and to perform the cut. The cut is perpendicular to the biomechanical axis **3700.** The first template **3705** has linkages or extenders **3710** for connecting a second template **3715** for the anterior cut **3702** and for connecting a third template **3720** for the posterior cut **3703.** The linkages **3710** connecting the first template **3705** with the second **3715** and third template **3720** help in achieving a reproducible position of the templates relative to each other. At least one of the templates, preferably the first template **3705,** will have a surface **3706** that is a mirror image of the articular surface **3708.** In this example, all three templates have surface facing the joint that is a mirror image of the joint, although one template having a surface conforming to the joint suffices in many applications.

A fourth template **3725** may optionally be used in order to perform an anterior chamfer cut **3704.** The fourth template may have a guide aperture or reference plane **3730** that can determine the anterior chamfer cut **3704.** The fourth template can, but must not have at least one surface **3735** matching one or more cut articular surfaces **3740.** The fourth template may have one or more outriggers or extenders **3745** stabilizing the template against the cut or uncut articular surface.

A fifth template **3750** may optionally be used to perform an anterior chamfer cut **3705.** The fifth template may have a guide aperture or reference plane **3755** that can determine the posterior chamfer cut **3705.** The fifth template may have at least one surface **3735** matching one or more cut articular surfaces **3740.** Oblique planes **3760** may help to further stabilize the template during the procedure. The fifth template may have one or more outriggers or extenders **3745** stabilizing the template against the cut or uncut articular surface.

In another embodiment, an opposite articular side **3765** may be cut in reference to a first articular side **3766.** Any order or sequence of cutting is possible: femur first then tibia, tibia first then femur, patella first, and so forth. A template **3770** may be shaped to the uncut or, in this example, cut first articular side. The template may have stabilizers against the first articular surface, for example with extenders **3772** into a previously created peg hole **3773** for an implant. The template may have a linkage or an extender **3775** to a second articular surface **3765.** Surgical instruments may be attached to the linkage or extender **3775.** In this example, a tibial cut guide **3778** with multiple apertures or reference planes **3779** for a horizontal tibial cut is attached. The tibial cut guide may but may not have a surface matching the tibial surface.

By referencing a first, e.g. femoral, to a second, e.g. tibial cut greater accuracy can be achieved in the alignment of these cuts, which will result in improved implant component alignment and less wear. Ratchet like devices **3785** or hinge like devices or spacers may be inserted into the space between the first and the second articular surface and soft-tissue tension and ligament balancing can be evaluated for different distances achieved between the first **3766** and second **3765** articular surface, with one or more of them being cut or uncut. In this manner, soft-tissue tension and ligament balancing can be tested during different pose angles, e.g. degrees of flexion or extension. Optionally, tensiometers can be used. Once an ideal soft-tissue tension and/or ligament balancing has been achieved, the tibial cut may be performed through one of the guide apertures **3779** in reference to the femoral cut.

### ii. 3D Guidance Molds for Ligament Repair and Replacement

3D guidance molds may also be utilized for planning the approach and preparing the surgical intervention and conducting the surgical intervention for ligament repair and replacement, in accordance with one embodiment.

In one example, the anterior cruciate ligament is replaced using a 3D guidance mold. The anterior cruciate ligament is a collagenous structure located in the center of the knee joint, and is covered by the synovial sheath. The ligament has an average length of thirty (30) to thirty-eight (38) millimeters and an average width of ten (10) to eleven (11) millimeters. The ligament is proximally attached to the posterior aspect of the lateral femoral condyle's medial surface. The ligament passes anteriorly, medially and distally within the joint to its attachment at the anteromedial region of the tibial plateau, between the tibial eminences. The distal portion of the ligament fans out to create a large tibial attachment known as the footprint of the ligament. The ligament has two functional subdivisions which include the anteromedial band and the posterolateral band. The posterolateral band is taut when the knee is extended and the anteromedial band becomes taut when the knee is flexed. Because of its internal architecture and attachments sides on femur and tibia, the ACL provides restraint to anterior translation and internal rotation of the tibia in angulation and hyperextension of the knee. The prevalence of ACL injuries are about 1 in 3,000 subjects in the United States and approximately 250,000 new injuries each year.

Other tendon and ligament injuries, for example, including the rotator cuff, the ankle tendons and ligaments, or the posterior cruciate ligament can also be highly prevalent and frequent.

Selecting the ideal osseous tunnel sights is a crucial step in ligament reconstruction, for example, the anterior and posterior cruciate ligament.

In the following paragraphs, embodiments will be described in detail as they can be applied to the anterior cruciate ligament. However, clearly all embodiments mentioned below and modifications thereof are applicable to other ligaments, including the posterior cruciate ligament and also tendons such as tendons around the ankle joint or rotator cuff and shoulder joint.

### Anterior Cruciate Ligament

The normal anterior cruciate ligament is composed of a large number of fibers. Each fiber can have a different length, a different origin and a different insertion and is frequently under different tension during the range of motion of the knee joint. One of the limitations of today's ACL graft is that they have parallel fibers. Thus, even with ideal selection of the placement of the osseous tunnels, fibers of an ACL graft will undergo length and tension changes with range of motion. Therefore, today's ACL replacement cannot duplicate the original ligament. However, placing the center of the osseous tunnels at the most isometric points, maximizes the stability that can be obtained during motion and minimizes later on graft wear and ultimately resultant failure.

In illustrative embodiments, 3D guidance templates may be selected and designed to enable highly accurate, reproducible and minimally invasive graft tunnels in the femur and the tibia.

In one embodiment, imaging such as MRI is performed pre-operatively. The images can be utilized to identify the origin of the ligament and its insertion onto the opposing articular surface, in the case of an anterior cruciate ligament, the tibia. Once the estimated location of the origin and the footprint, i.e. the insertion of the ligament has been identified, 3D guidance templates may be made to be applied to these areas or their vicinity.

The 3D guidance templates may be made and shaped to the articular surface, for example, adjacent to the intended tunnel location or they may be shaped to bone or cartilage outside the weight bearing zone, for example, in the intercondylar notch. A 3D guidance template for femoral or tibial tunnel placement for ACL repair may include blocks, attachments or linkages for reference points or guide aperture to guide and direct the direction and orientation of a drill, and optionally, also the drill depth. Optionally, the 3D guidance templates may be hollow. The 3D guidance templates may be circular, semi-circular or ellipsoid. The 3D guidance templates may have a central opening to accommodate a drill.

In one embodiment, the 3D guidance template is placed on, over or near the intended femoral or tibial entry point and subsequently the drill hole. Once proper anatomic positioning has been achieved, the ligament tunnel can be created. The 3D guidance template, its shape, position, and orientation, may be optimized to reflect the desired tunnel location in the femur and the tibia, wherein the tunnel location, position, orientation and angulation is selected to achieve the best possible functional results. Additional considerations in placing the femoral or tibial tunnel includes a sufficient distance to the cortical bone in order to avoid failure or fracture of the tunnel.

Thus, optionally, the distance of the tunnel to the adjacent cortical bone and also other articular structures may optionally be factored into the position, shape and orientation of the femoral or tibial 3D guidance templates in order to achieve the optimal compromise between optimal ligament function and possible post-operative complications such as failure of the tunnel.

In another embodiment, the imaging test may be utilized to determine the origin and insertion of the ligament. This determination can be performed on the basis of bony landmarks identified on the scan, *e.g.* a CT scan or MRI scan. Alternatively, this determination can be performed by identifying ligament remnants, for example, in the area of the ligament origin and ligament attachment. By determining the origin and the insertion of the ligament the intended graft length may be estimated and measured. This measurement may be performed for different pose angles of the joint such as different degrees of flexion, extension, abduction, adduction, internal and external rotation.

In another embodiment, the imaging test may be utilized to identify the ideal graft harvest site wherein the graft harvest site can optionally be chosen to include sufficiently long ligament portion and underlying bone block proximally and distally in order to fulfill the requirement for graft length as measured earlier in the imaging test. An additional 3D guidance template for the same 3D guidance templates, possibly with linkages, may be utilized to harvest the ligament and bone from the donor site in the case of an autograft. Optionally, 3D guidance templates may also be utilized or designed or shaped or selected to guide the extent of an optional notchplasty. This can include, for example, the removal of osteophytes.

In the case of an ACL replacement, the 3D guidance templates may in this manner optimize selection of femoral and tibial tunnel sites. Tunnel sites may even be optimized for different knee pose angles, i.e. joint positions, and different range of motion. Selecting the properly positioned femoral tunnel site ensures maximum post operative knee stability.

The intra-articular site of the tibial tunnel has less effect on changes in graft length but its position can be optimized using proper placement, position, and shape of 3D guidance templates to prevent intercondylar notch impingement.

Moreover, the 3D guidance templates may include an optional stop for the drill, for example, to avoid damage to adjacent neurovascular bundles or adjacent articular structures, including the articular cartilage or other ligaments.

Optionally, the 3D guidance templates may also include a stop, for example, for a drill in order to include the drill depth.

The direction and orientation of the tibial tunnel and also the femoral tunnel may be determined with use of the 3D guidance template, whereby it will also include selection of an optimal tunnel orientation in order to match graft length as measured pre-operatively with the tunnel length and the intra-articular length of the graft ligament.

In one embodiment, a tibial 3D guidance template is, for example, selected so that its opening is located immediately posterior to the anatomic center of the ACL tibial footprint. Anatomic landmarks may be factored into the design, shape, orientation, and position of the tibial guidance template, optionally. These include, without limitation, the anterior horn of the lateral meniscus, the medial tibial spine, the posterior cruciate ligament, and the anterior cruciate ligament stump.

The tunnel site may be located utilizing the 3D guidance template in the anterior posterior plane by extending a line in continuation with the inner edge of the anterior horn of the lateral meniscus. This plane will typically be located six (6) to seven (7) millimeters anterior to the interior border of the PCL. The position, shape and orientation of the 3D guidance template will be typically so that the resultant tibial tunnel and the resultant location and orientation of the ACL graft, once in place, may touch the lateral aspect of the PCL, but will not significantly deflect it. Similarly, the location of the tibial guidance template and the resultant ligament tunnel and the resultant location of the ACL graft, once in place, may be chosen so that the graft will neither abrade nor impinge against the medial aspect of the lateral femoral condyle or the roof of the intercondylar notch when the knee is, for example, in full extension. In this manner, highly accurate graft placement is possible thereby avoiding the problems of impingement and subsequent graft failure.

In another embodiment, the pre-operative scan can be evaluated to determine the maximal possible graft length, for example, patella tendon graft. If there is concern that the maximal graft length is not sufficient for the intended ACL replacement, the tunnel location and orientation, specifically the exits from the femur or the tibia can be altered and optimized in order to match the graft length with the tunnel length and intra-articular length.

In a preferred embodiment, the graft length is measured or simulated pre-operatively, for example, by measuring the optimal graft length for different flexion and extension angles. Using this approach, an optimal position, shape, orientation and design of the 3D guidance template may be derived at an optimal compromise between isometric graft placement, avoidance of impingement onto the PCL, and/or avoidance of impingement onto the femoral condyle, maximizing achievable graft lengths.

Intraoperatively, the femoral and/or tibial 3D guidance templates may include adjustment means. These adjustment means can, for example, allow movement of the template by one or two or more millimeters intervals in posterior or medial or lateral orientation, with resultant movement of the femoral or tibial tunnel. Additionally, intraoperative adjustment may also allow for rotation of the template, with resultant rotation of the resultant femoral or tibial tunnels.

A single template may be utilized to derive the femoral tunnel. A single template may also be utilized to derive the tibial tunnel. More than one template may be used on either side.

Optionally, the templates may include linkages, for example, for attaching additional measurement devices, guide wires, or other surgical instruments. Alignment guides including mechanical, electrical or optical devices may be attached or incorporated in this manner.

In another embodiment, the opposite articular surface may be cross referenced against a first articular surface. For example, in the case of an ACL repair, the femoral tunnel may be prepared first using a 3D guidance template, whereby the 3D guidance template helps determine the optimal femoral tunnel position, location, orientation, diameter, and shape. The femoral guidance template may include a link inferiorly to the tibia or an attachable linkage, wherein said link or said attachable linkage may be utilized to determine the ideal articular entry point for the tibial tunnel. In this manner, the tibial tunnel can be created in an anatomic environment and in mechanical cross reference with the femoral tunnel. The reverse approach is possible, whereby the tibial tunnel is created first using the 3D guidance template with a link or linkage to a subsequently created femoral tunnel. Creating the femoral or tibial tunnel in reference to each other advantageously helps reduce the difficulty in performing the ligament repair and also can improve the accuracy of the surgery in select clinical situations.

In another embodiment, the template for ligament repair may include optional flanges or extenders. These flanges or extenders may have the function of tissue retractors. By having tissue retractor function, the intra-articular template for ligament repair can provide the surgeon with a clearer entry to the intended site of surgical intervention and improve visualization. Moreover, flanges or extenders originating from or attached to the 3D guidance templates may also serve as tissue protectors, for example, protecting the posterior cruciate ligament, the articular cartilage, or other articular structures as well as extra-articular structures.

In another embodiment, an additional 3D guidance template or linkages to a first or second articular 3D guidance templates can be utilized to place ligament attachment means, for example, interference crews.

If an allograft is chosen and the allograft length and optionally, dimensions are known pre-operatively, additional adjustments may be made to the position, shape and orientation of the 3D guidance templates and additional tunnels in order to match graft dimensions with tunnel dimensions and graft length with intra-femoral tunnel length, intra-articular length and intra-tibial tunnel length. Optionally, this adjustment and optimization can be performed for different pose angles of the joint, *e.g.* different degrees of flexion or extension.

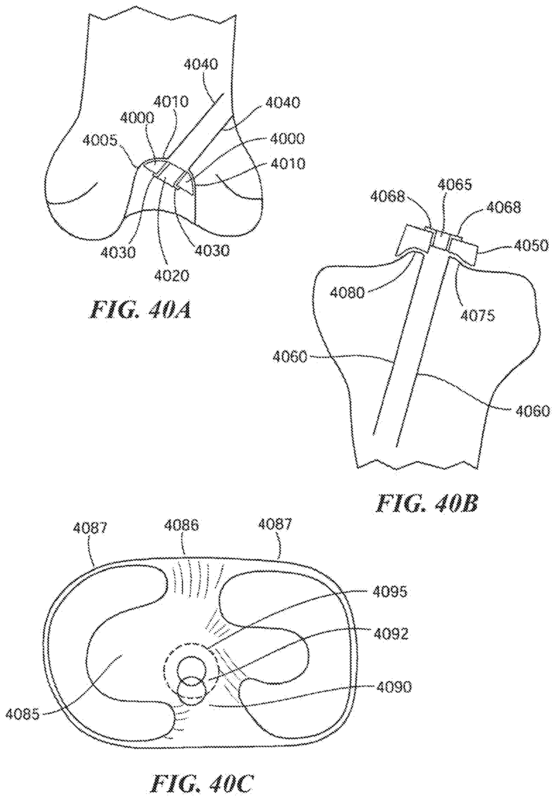

**FIGS. 40A-C** illustrate an exemplary use of 3D guidance templates for performing ligament repair; in this case repair of the anterior cruciate ligament (ACL). A 3D guidance template **4000** is placed in the intercondylar notch region **4005.** At least one surface **4010** of the template **4000** is a mirror image of at least portions of the notch **4005** or the femur. The template **4000** may be optionally placed against the trochlea and/or the femoral condyle (not shown). The mold **4000** includes an opening **4020** and, optionally, metal sleeves **4030,** wherein the position, location and orientation of the opening **4020** and/or the metal sleeves **4030** determine the position and orientation of the femoral graft tunnel **4040.**

A tibial template **4050** may be used to determine the location and orientation of the tibial tunnel **4060.** Specifically, an opening **4065** within the tibial mold **4050** will determine the position, angle and orientation of the tibial tunnel **4060.** The opening may include optional metal sleeves **4068.** At least one surface **4070** of the tibial template **4050** will substantially match the surface of the tibia **4075.** The template may be matched to a tibial spine **4080** wherein the tibial spine can help identify the correct position of the mold and help fix the template in place during the surgical intervention. Of note, the sleeves **4030** and **4068** may be made of other hard materials, e.g. ceramics. The femoral and/or tibial template may be optionally attached to the femoral or tibial articular surface during the procedure, for example using K-wires or screws.

**FIG. 40C** shows a top view of the tibial plateau **4085.** The PCL **4086** is seen as are the menisci **4087.** The original site of ACL attachment **4090** is shown. The intended tunnel site **4092** may be slightly posterior to the original ACL attachment **4090.** The template **4095** may placed over the intended graft tunnel **4092.** The template will typically have a perimeter slightly greater than the intended tunnel site. The templates may allow for attachments, linkages or handles.

### PCL Repair

All of the embodiments described above may also be applied to PCL repair as well as the repair of other ligaments or tendons.

For PCL repair, 3D guidance templates may be designed for single, as well as double bundle surgical technique. With single bundle surgical technique, a 3D guidance template may be created with a position, orientation and shape of the template or associated reference points or guide apertures for surgical instruments that will help create a femoral tunnel in the location of the anatomic origin of the ligament. Alternatively, the template and any related reference points or guide apertures or linkages may be designed and placed so that an anterior placement of the femoral tunnel in the anatomic footprint is performed. A more anterior placement of the femoral tunnel can restore normal knee laxity better than isometric graft placement. The 3D guidance templates may be designed so that optimal tension is achieved not only in knee extension but also in knee flexion, particularly ninety degrees of knee flexion. Thus, the origin and the insertion of the PCL may be identified pre-operatively on the scan, either by identifying residual fiber bundles or by identifying the underlying anatomic landmarks. The distance between the origin and the insertion may thus be determined in the extension and can be simulated for different flexion degrees or other articular positions. Femoral and tibial tunnel placement and orientation may then be optimized in order to achieve an isometric or near isometric ligament placement. Intraoperative adjustments are feasible as described in the foregoing embodiments.

A 3D guidance template may also be designed both on the femoral as well as on the tibial side using double bundle reconstruction techniques. With double bundle reconstruction techniques, the femoral or tibial template can include or incorporate links or can have attachable linkages so that a femoral tunnel can be created and cross referenced with a tibial tunnel, or a tibial tunnel can be created and cross referenced to a femoral tunnel.

As described for the ACL, the templates may include stops for drills and reaming devices or other surgical instruments, for example, to protect popliteal neurovascular structures. The templates may include extenders or flanges to serve as tissue retractors as well as tissue protectors.

In principle, templates may be designed to be compatible with any desired surgical technique. In the case of PCL repair, templates may be designed to be compatible with single bundle, or a double bundle reconstruction, tibial inlay techniques as well as other approaches.

As previously stated, 3D guidance templates are applicable to any type of ligament or tendon repair and can provide reproducible, simple intraoperative location of intended attachment sites or tunnels. The shape, orientation and position of the 3D guidance templates may be individualized and optimized for articular anatomy, as well as the biomechanical situation, and may incorporate not only the articular shape but also anatomic lines, anatomic planes, biomechanical lines or biomechanical planes, as well as portions or all of the shape of devices or anchors or instruments to be implanted or to be used during implantation or to be used during surgical repair of a ligament or tendon tear.

### D. Surgical Navigation and 3D Guidance Templates

3D guidance template technology as described herein may be combined with surgical navigation techniques. Surgical navigation techniques may be image guided or non-image guided for this purpose. Passive or active surgical navigation systems may be employed. Surgical navigation systems that use optical or radiofrequency transmission or registration may be used. A representative example is the Vector Vision navigation system manufactured by Brain Lab, Germany. This is a passive infrared navigation system. Once the patient is positioned appropriately in the operating room, optical, e.g. retro-reflective, or radiofrequency markers can be applied to the extremity near the area of intended surgery. With image guided navigation, an imaging study such as a CT scan or MRI scan, can be transferred into the workstation of the navigation system. For registration purposes, the surgeon can, for example, utilize a pointer navigation tool to touch four or more reference points that are simultaneously co-identified and cross registered on the CT or MRI scan on the workstation. In the knee joint, reference points may include the trochlear groove, the most lateral point of the lateral condyle, the most medial femoral condyle, the tip of the tibial spines and so forth. Using image guided navigation, anatomical and biomechanical axis of the joint can be determined reliably.

Alternatively, non-image guided navigation may be utilized. In this case, optical, e.g. retro-reflective, markers or small radio frequency transmitters are positioned on the extremity. Movement of the extremity and of the joints is utilized, for example, to identify the center of rotation. If surgery of the knee joint is contemplated, the knee joint may be rotated around the femur. The marker or radiofrequency transmitter motion may be utilized to identify the center of the rotation, which will coincide with the center of the femoral head. In this manner, the biomechanical axis may be determined non-invasively.

The information resulting in imaging guided navigation, pertaining to either anatomical or biomechanical axis can be may be utilized to optimize the position of any molds, blocks, linkages or surgical instruments attached to or guided through the 3D guidance molds.

In one embodiment, the joint or more specifically the articular surface, may be scanned intra-operatively, for example, using ultrasound or optical imaging methods. The optical imaging methods may include stereographic or stereographic like imaging approaches, for example, multiple light path stereographic imaging of the joint and the articular surface or even single light path 3D optical imaging. Other scan technologies that are applicable are, for example, C-arm mounted fluoroscopic imaging systems that can optionally also be utilized to generate cross-sectional images such as a CT scan. Intraoperative CT scanners are also applicable. Utilizing the intraoperative scan, a point cloud of the joint or the articular surface or a 3D reconstruction or a 3D visualization and other 3D representations may be generated that can be utilized to generate an individualized template wherein at least a portion of said template includes a surface that is a mirror image of the joint or the articular surface. A rapid prototyping or a milling or other manufacturing machine can be available in or near the operating room and the 3D guidance template may be generated intraoperatively.

The intraoperative scan in conjunction with the rapid production of an individualized 3D guidance template matching the joint or the articular surface, in whole or at least in part, has the advantage to generate rapidly a tool for rapid intraoperative localization of anatomical landmarks, including articular landmarks. A 3D guidance template may then optionally be cross-registered, for example, using optical markers or radiofrequency transmitters attached to the template with the surgical navigation system. By cross-referencing the 3D guidance template with the surgical navigation system, surgical instruments can now be reproducibly positioned in relationship to the 3D guidance template to perform subsequent procedures in alignment with or in a defined relationship to at least one or more anatomical axis and/or at least one or more biomechanical axis or planes.

### E. Stereoscopy, Stereoscopic Imaging

In addition to cross-sectional or volumetric imaging technologies including CT, spiral CT, and MRI, stereoscopic imaging modalities may be utilized. Stereoscopic imaging is any technique capable of recording three-dimensional information from two two-dimensional, projectional imaging. Traditional stereoscopic imaging includes creating a 3D visualization or representation starting from a pair of 2D images. The projection path of the 2D images is offset. The offset is, for example, designed to create an impression of object depth for the eyes of the viewer. The offset or minor deviation between the two images is similar to the prospectors that both eyes naturally receive in binocular vision. Using two or more images with an offset or minor deviation in perspective, it is possible to generate a point cloud or 3D surface or 3D visualization of a joint or an articular surface, which can then be input into a manufacturing system such as a rapid prototyping or milling machine. Dual or more light path, as well as single light path, systems can be employed

### F. Knee Joint

When a total knee arthroplasty is contemplated, the patient can undergo an imaging test, as discussed in more detail above, that will demonstrate the articular anatomy of a knee joint, *e.g.* width of the femoral condyles, the tibial plateau etc. Additionally, other joints can be included in the imaging test thereby yielding information on femoral and tibial axes, deformities such as varus and valgus and other articular alignment. The imaging test can be an x-ray image, preferably in standing, load-bearing position, a CT or spiral CT scan or an MRI scan or combinations thereof. A spiral CT scan may be advantageous over a standard CT scan due to its improved spatial resolution in z-direction in addition to x and y resolution. The articular surface and shape as well as alignment information generated with the imaging test can be used to shape the surgical assistance device, to select the surgical assistance device from a library of different devices with pre-made shapes and sizes, or can be entered into the surgical assistance device and can be used to define the preferred location and orientation of saw guides or drill holes or guides for reaming devices or other surgical instruments. Intraoperatively, the surgical assistance device is applied to the tibial plateau and subsequently the femoral condyle(s) by matching its surface with the articular surface or by attaching it to anatomic reference points on the bone or cartilage. The surgeon can then introduce a reamer or saw through the guides and prepare the joint for the implantation. By cutting the cartilage and bone along anatomically defined planes, a more reproducible placement of the implant can be achieved. This can ultimately result in improved postoperative results by optimizing biomechanical stresses applied to the implant and surrounding bone for the patient's anatomy and by minimizing axis malalignment of the implant. In addition, the surgical assistance device can greatly reduce the number of surgical instruments needed for total or unicompartmental knee arthroplasty. Thus, the use of one or more surgical assistance devices can help make joint arthroplasty more accurate, improve postoperative results, improve long-term implant survival, reduce cost by reducing the number of surgical instruments used. Moreover, the use of one or more surgical assistance device can help lower the technical difficulty of the procedure and can help decrease operating room ("OR") times.

Thus, surgical tools described herein can also be designed and used to control drill alignment, depth and width, for example when preparing a site to receive an implant. For example, the tools described herein, which typically conform to the joint surface, can provide for improved drill alignment and more accurate placement of any implant. An anatomically correct tool can be constructed by a number of methods and can be made of any material, preferably a substantially translucent and/or transparent material such as plastic, Lucite, silastic, SLA or the like, and typically is a block-like shape prior to molding.

**FIG. 24A** depicts, in cross-section, an example of a mold ***600*** for use on the tibial surface having an upper surface ***620.*** The mold ***600*** contains an aperture ***625*** through which a surgical drill or saw can fit. The aperture guides the drill or saw to make the proper hole or cut in the underlying bone **610** as illustrated in **FIGS. 21B-D****.** Dotted lines ***632*** illustrate where the cut corresponding to the aperture will be made in bone.

**FIG. 24B** depicts, a mold ***608*** suitable for use on the femur. As can be appreciated from this perspective, additional apertures are provided to enable additional cuts to the bone surface. The apertures ***605*** enable cuts ***606*** to the surface of the femur. The resulting shape of the femur corresponds to the shape of the interior surface of the femoral implant, typically as shown in **FIG. 21E****.** Additional shapes can be achieved, if desired, by changing the size, orientation and placement of the apertures. Such changes would be desired where, for example, the interior shape of the femoral component of the implant requires a different shape of the prepared femur surface.

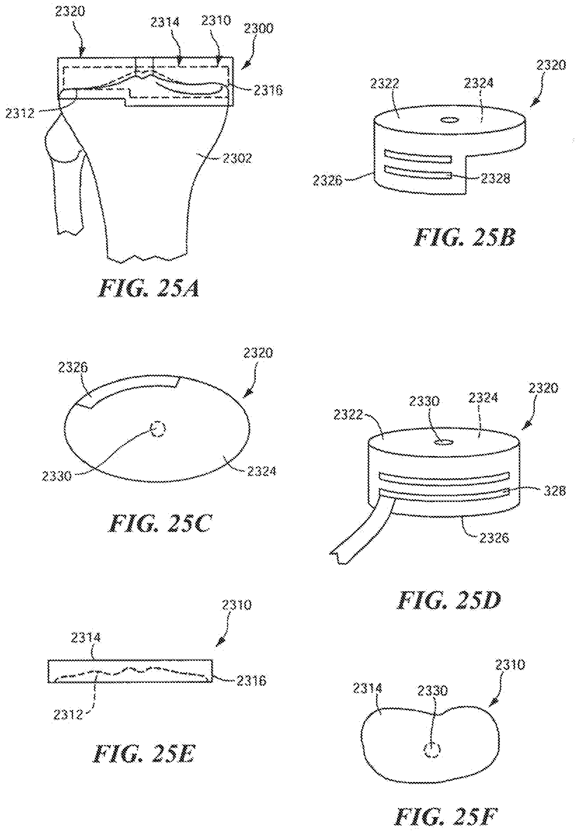

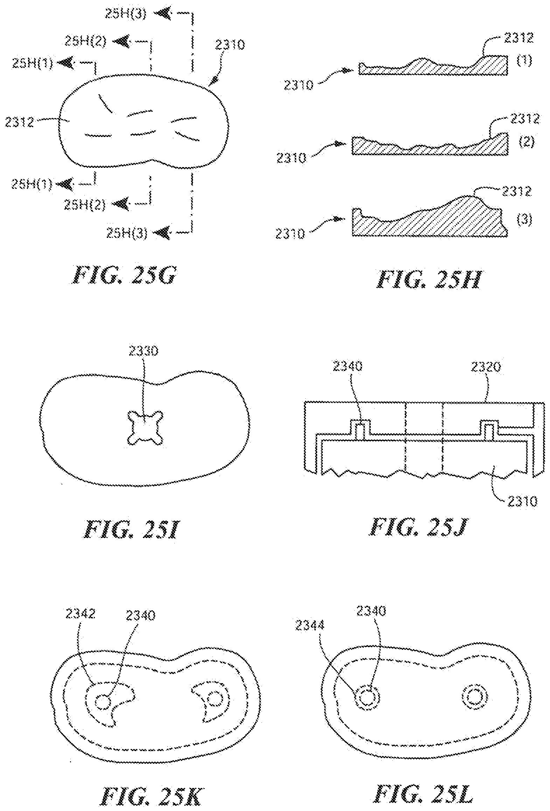

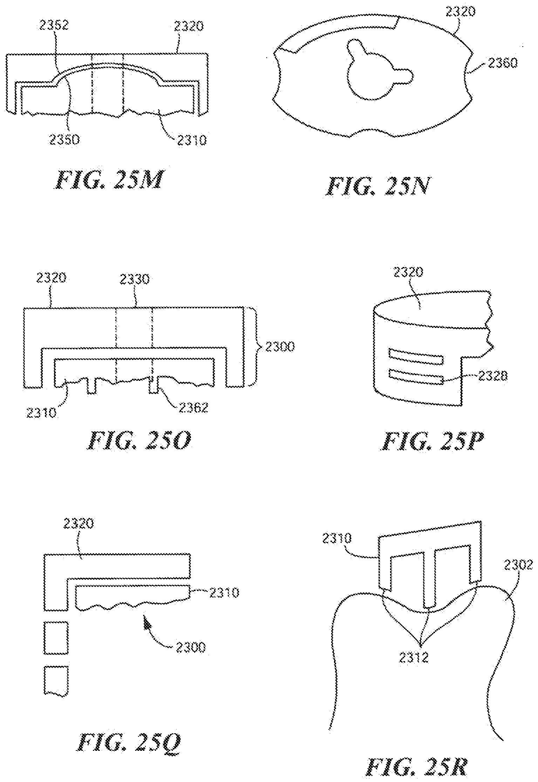

Turning now to **FIG. 25****,** a variety of illustrations are provided showing a tibial cutting block and mold system. **FIG. 25A** illustrates the tibial cutting block ***2300*** in conjunction with a tibia ***2302*** that has not been resected. In this depiction, the cutting block ***2300*** consists of at least two pieces. The first piece is a patient specific interior piece ***2310*** or mold that is designed on its inferior surface ***2312*** to mate, or substantially mate, with the existing geography of the patient's tibia ***2302.*** The superior surface ***2314*** and side surfaces ***2316*** of the first piece ***2310*** are configured to mate within the interior of an exterior piece ***2320.*** The reusable exterior piece ***2320*** fits over the interior piece ***2310.*** The system can be configured to hold the mold onto the bone.

The reusable exterior piece has a superior surface ***2322*** and an inferior surface ***2324*** that mates with the first piece ***2310.*** The reusable exterior piece ***2320*** includes cutting guides ***2328,*** to assist the surgeon in performing the tibial surface cut described above. As shown herein a plurality of cutting guides can be provided to provide the surgeon a variety of locations to choose from in making the tibial cut. If necessary, additional spacers can be provided that fit between the first patient configured, or molded, piece ***2310*** and the second reusable exterior piece, or cutting block, ***2320.***

Clearly, the mold may be a single component or multiple components. In a preferred embodiment, one or more components are patient specific while other components such as spacers or connectors to surgical instruments are generic. In one embodiment, the mold can rest on portions of the joint on the articular surface or external to the articular surface. Other surgical tools then may connect to the mold. For example, a standard surgical cut block as described for standard implants, for example in the knee the J&J PFC Sigma system, the Zimmer Nexgen system or the Stryker Duracon system, can be connected or placed on the mold. In this manner, the patient specific component can be minimized and can be made compatible with standard surgical instruments.

The mold may include receptacles for standard surgical instruments including alignment tools or guides. For example, a tibial mold for use in knee surgery may have an extender or a receptacle or an opening to receive a tibial alignment rod. In this manner, the position of the mold can be checked against the standard alignment tools and methods. Moreover, the combined use of molds and standard alignment tools including also surgical navigation techniques can help improve the accuracy of or optimize component placement in joint arthroplasty, such as hip or knee arthroplasty. For example, the mold can help define the depth of a horizontal tibial cut for placement of a tibial component. A tibial alignment guide, for example an extramedullary or intramedullary alignment guide, used in conjunction with a tibial mold can help find the optimal anteroposterior angulation, posterior slope, tibial slant, or varus-valgus angle of the tibial cut. The mold may be designed to work in conjunction with traditional alignment tools known in the art.

The mold may include markers, e.g. optoelectronic or radiofrequency, for surgical navigation. The mold may have receptacles to which such markers can be attached, either directly or via a linking member.

The molds can be used in combination with a surgical navigation system. They can be used to register the bones associated with a joint into the coordinate system of the surgical navigation system. For example, if a mold for a joint surface includes tracking markers for surgical navigation, the exact position and orientation of the bone can be detected by the surgical navigation system after placement of the mold in its unique position. This helps to avoid the time-consuming need to acquire the coordinates of tens to hundreds of points on the joint surface for registration.

Any marker known in the art or developed in the future can be used for surgical navigation or robotic surgery. Such markers include radiofrequency devices or optical devices. Markers can be attached to: 1. Skin; 2. Bone, e.g. via fixation pins drilled into bone; 3. Molds or custom/patient specific jigs for creating, for example, a drill hole or a cut; 4. Standard instruments, e.g. referenced to a drill hole or a cut surface (optionally created with use of a patient specific mold) or attached to a patient specific mold.

As outlined above, by attaching the markers to the mold, the need for registration of the markers in space may be obviated or minimized.

In one embodiment, the molds are used for referencing of anatomical landmarks or anatomical or biomechanical axes. Optionally one or more surgical steps can be performed using the molds, e.g. a distal femoral cut or a proximal tibial cut. Markers can be attached to skin, bone or one or more molds as well as any patient specific or standard instruments, optionally attached to a mold or linked to or referenced to the patient's joint or articular surface or an altered surface, e.g. a cut surface or a drill hole made with a patient specific mold. Markers can be used on a femur or a tibia or both, attached to one or more of the tissues or devices mentioned above. In other joints, e.g. a hip, a shoulder joint, an ankle joint, an elbow, a spine, markers can be used on an acetabulum or a proximal femur, or both, a glenoid or a proximal humerus, or both, a talus or calcaneus or a distal tibia, or combinations thereof, a radius or ulna or distal humerus, or combinations thereof, a first vertebral body, a second vertebral body, vertebral joints, or combinations thereof, attached to one or more tissues or devices mentioned above.

With the markers in place, the joint, e.g. a knee, hip, shoulder, ankle, elbow, or spine can be moved through a range of motion, e.g. a flexion or extension, an internal or external rotation, an adduction or abduction, an elevation. Any degree of motion is possible. The joint can be moved at different speeds, for one or more directional movements, and marker motion can be captured at different speeds. The joint can be moved passively, e.g. during surgery under anesthesia, or actively, e.g. by the patient, e.g. prior to surgery with markers, for example, attached to the skin. Preoperative and intraoperative marker movement and related measurements can be compared, optionally before or after performing any surgical steps. Pre- and intraoperative measurements can also be obtained during stress testing, e.g. with use of weights or mechanical actuators, e.g. a KT1000 system or similar machine for evaluating the anterior cruciate ligament.

Any of the measurements can be obtained pre-operatively or intraoperatively prior to performing any surgical steps, or with one or more surgical steps already performed, e.g. a cutting or a drilling. Measurements of joint motion can be compared, e.g. Preoperative vs. intraoperative before surgical steps; Preoperative vs. intraoperative after surgical steps; Intraoperative before vs. intraoperative after surgical steps.

Any of the above measurements can be compared to reference databases of joint motion. These reference databases can include patient demographic information including, but not limited to, age, gender, height, weight, BMI, activity level, limb circumference etc. The reference database can also include anatomic and biomechanical data, optionally grouped into different classes. The reference database can include model kinematic or biomotion data for the unoperated joint, e.g. a hip, knee, ankle, shoulder, elbow, wrist joint or a spine. The reference database can also include model kinematic or biomotion data for the joint in an operated condition. For example, in the knee, the reference database can include kinematic or biomotion data after placement of a particular type of knee implant, e.g. a posterior cruciate retaining, a posterior stabilized or a bi-cruciate retaining knee implant, as well as partial knee implants. These databases can include reference kinematic or biomotion patterns or data for a particular implant type, size or shape. The database can also include different shapes available for a given implant type.

Optionally, the patient can undergo a pre-operative scan, e.g. a CT scan, MRI scan or ultrasound scan or fluoroscopic scan, with or without contrast. The scan can be used to generate a patient specific template as described in other embodiments. The scan can also be used to determine select anthropometric, anatomic or biomechanical or kinematic features of a patient. These features can, for example, include the location and orientation of certain anatomic or biomechanical axes, the curvature of the joint, e.g. the sagittal curvature of the femoral condyle(s) in a knee joint, a tibial slope, e.g. of a medial or a lateral tibial plateau or both, and, principally any anatomic or biomechanical information relevant to the patient's surgery, the implant position, and the desired kinematic outcome. Using one or more of these patient specific features obtained from the pre-operative scan, a desirable postoperative biomotion or kinematic pattern can be selected from the reference database. In joint reconstruction, a desired implant can be selected matching the implant, for example to 1) An AP dimension; 2) An ML dimension; 3) An SI dimension; 4) A curvature of the joint.

The kinematic model can include muscle simulations including muscle activation and ligament simulations. The muscle data and/or ligament data can be selected from a pre-existing database. Alternatively, the patient's scan data can be used to introduce muscle data or ligament data of the patient or combinations thereof. For example, the location of a muscle, its width and volume can be introduced into the kinematic model, for example for purposes of estimating muscle strength and forces. The moment arms can be determined based on the location of the muscles and their tendons. Tendon location, width, length, thickness can be introduced into the model, for example derived from the patient's scan data. Tendons can be directly visualized on the scan and segmented and introduced into the model. Alternatively, the tendon origin and insertion can be identified on the scan and can be used for kinematic modeling.

The implant position and orientation can be adjusted in the kinematic model to achieve a desired post-implantation kinematic or biomotion pattern or performance. Bone cuts or reaming or drilling or other surgical interventions can be simulated and can be adjusted to change the implant position, for example in a knee joint, a hip joint or a shoulder joint. The adjustment or optimization of the implant position and orientation and any related surgical interventions can be performed manually, with optionally re-assessment of the kinematic or biomotion pattern or performance after adjustment. The adjustment or optimization of the implant position and orientation and any related surgical interventions can also be performed automatically or semi-automatically, e.g. with optional manual user interaction or input. By utilizing the patient's anatomic information to select an implant and by optionally utilizing the patient's demographic, anatomic, axis, biomechanical and/or kinematic information it is possible to optimize implant placement / position and orientation on one or more articular sides thereby improving the postoperative kinematic result. In one embodiment, the optimizations will be focused towards achieving a postoperative, e.g. post implantation, condition for a given patient that will result in a natural or near natural state of joint kinematics or biomotion similar to a health, un-operated state.

The model with the implant included can also include information about the patient's bone shape, cartilage shape, articular curvatures, slopes as well as ligament and muscle information.

The implant position or orientation can be adjusted by adjusting the position of one or more patient specific molds or by adjusting the position of drill guides or cut guides or other guides within these molds or attached to these molds, thereby adjusting the implant position or orientation. Exemplary parameters of implant position or orientation that can be influenced or optimized in this manner based on the database, pre-operative scan measurements, scan data, as well as intraoperative measurements include, but are not limited to: Implant position, e.g. AP, ML, SI; Implant position to avoid notching, e.g. in knee implants; Implant orientation; Implant rotation, e.g. internal or external; Implant flexion; Implant extension; Implant anteversion; Implant retroversion; Implant abduction; Implant adduction; Implant joint line, e.g. between a femoral component and a tibial component.

Optionally, the joint can be moved through a range of motion with a trial implant in place or the definitive implant in place, but not permanently affixed yet to the joint. Measurements can thus be obtained:

| | |
|---|---|
| 1. Preoperative | a. Active |
| | b. Passive |
| | c. With optional stress testing |
| 2. Intraoperative prior to performing surgical steps, i.e. on the unaltered joint | a. Active, e.g. before anesthesia |
| | b. Passive |
| | c. Passive with optional stress testing |
| 3. Intraoperative after performing surgical steps | a. Passive |
| | b. Passive with optional stress testing |
| 4. Intraoperative with trial implant in place | a. Passive |
| | b. Passive with optional stress testing |
| 5. Intraoperative with definitive implant in place, not affixed yet | a. Passive |
| | b. Passive with optional stress testing |
| 6. Intraoperative with definitive implant in place, affixed to joint/bone | a. Passive |
| | b. Passive with optional stress testing |

Optionally, the same or similar measurements can be obtained for the contralateral joint, pre-operatively or intraoperatively.

Markers can be attached preoperatively or intraoperatively. The surgical navigation system can capture marker motion during the motion of the joint. Similarly, a robot can be used to monitor marker motion and joint motion.

By measuring marker motion, e.g. directly or indirectly attached or linked to an extremity, e.g. a femur or tibia, or a spine, preoperatively or intraoperatively prior to performing surgical steps or with only a few initial surgical steps performed, joint kinematics can be assessed. The position or orientation of a mold can then optionally be adjusted as a means of adjusting the position or orientation of the implant after placement in order to achieve a better or more desired kinematic result. The position or orientation of a guide within a mold can then optionally be adjusted as a means of adjusting the position or orientation of the implant after placement in order to achieve a better or more desired kinematic result. The position or orientation of both a mold and a guide within a mold can then optionally be adjusted as a means of adjusting the position or orientation of the implant after placement in order to achieve a better or more desired kinematic result. Such an improved or desired kinematic result can include one or more of 1) improvements in ligament balancing, e.g. optimization of flexion and extension gap or balancing; 2) improvements in range of motion, e.g. flexion and extension; 3) improvements in joint stability, e.g. as a means of reducing the possibility of subluxation or dislocation; 4) improvements in performance for select daily activities, e.g. stair climbing or going downstairs; 5) Avoidance or reduction of well know problems with joint replacement, e.g. mid-flexion instability.

Thus, the illustrative embodiments allow one to measure joint motion prior to implantation, e.g. pre-operatively or intra-operatively, or after performing select surgical steps. Preoperative, e.g. via a virtual simulation of joint kinematics optionally including patient data including scan data, and intraoperative measurements can include measurements of one or more dimensions of the joint, e.g. in an AP, ML, SI or oblique planes, one or more curvatures of the joint, e.g. of cartilage or subchondral bone, one or more slopes of the joint, measurements of distances, e.g. from a medial to a lateral condyle, e.g. a condylar length or height, width of a notch, and measurements or estimations of ligaments, ligament locations, strength, insertion, origin, muscle location, strength, insertion, origin and the like. Any of these simulations, both pre-operatively and intraoperatively, can also include finite element modeling, for example for estimating the stress or forces exerted on an implant, e.g. in select implant locations or along a chamfer cut. The finite element data can be augment with patient specific data, e.g. data obtained from the patient's scan including also for example bone mineral density or structure or any of the parameters mentioned above and throughout the specification.

If kinematic optimizations are simulated pre-operatively, they can be used to adjust the position or orientation of a mold or guide or combinations thereof used during surgery. This can, optionally, result in a change of the physical shape of the guide or the mold. If kinematic measurements are performed during the surgical procedure, for example by measuring marker motion during a range of motion prior to placing an implant, the position or orientation of a patient specific mold or guide included therein or attached thereto can be adjusted intraoperatively. Such adjustments can be, for example, performed with use of shims, spacers, spacer blocks, ratchet like mechanisms, dial-like mechanisms, electronic mechanisms, and other mechanisms known in the art or developed in the future. Alternatively, the mold can include more than one guide so that the position of a drill hole, a peg hole or a cut can be adjusted intraoperativly. Alternatively, the mold can allow for attachment of a block, e.g. for drilling or cutting, either in multiple different locations for kinematic optimization, or the position of the block can be adjusted by inserting, for example, shims or spacers between the mold and the block.

Thus, while patient specific molds will typically place an implant in a fixed position and orientation, for example relative to one or more anatomic or biomechanical axes or anatomic landmarks, the methods described herein allow for optimization of implant position for a desired, improved kinematic result.

Possible adjustments include one or more of: 1) Adjustment of implant flexion (or extension) relative to one or more anatomic or biomechanical axes, e.g. femoral component flexion in a knee prosthesis; 2) Adjustment of implant rotation (e.g. internal or external) relative to one or more anatomic or biomechanical axes or landmarks, e.g. femoral component rotation for flexion and/or extension balancing, or tibial component rotation; 3) Adjustment of anterior or posterior implant position, e.g. femoral component position (e.g. for flexion balancing) or tibial component position relative to one or more anatomic or biomechanical axes or landmarks; 4) Adjustment of medial or lateral implant position, e.g. femoral component position or tibial component position relative to one or more anatomic or biomechanical axes or landmarks; 5) Adjustment of superior or inferior implant position, e.g. femoral component position or tibial component position relative to one or more anatomic or biomechanical axes or landmarks (optionally performed via recuts).

The adjustments can include that an AP cut guide placed on a distal femur is rotated in order to rotate the implant position. A flexion spacer or cut guide can be rotated or changed in position, for example with a spacer or shim, in order to change implant position or orientation, for example for flexion balancing. A tibial guide can be rotated, for example for controlling varus or valgus or for controlling tibial component rotation.

### G. Ultrasound

In another embodiment, an ultrasound scan can be obtained. The ultrasound scan can be obtained in 1D, 2D and 3D. The scan can include information about the curvature of the joint, e.g. a cartilage or subchondral bone, and its surface shape. This information can be used to generate a patient specific jig with at least one portion including a patient specific surface derived from the scan.

The one or more ultrasound transducers can optionally be mounted on a holding apparatus. The position of the holding apparatus can optionally be registered relative to the joint. Alternatively, the ultrasound images or data can be registered intraoperatively by using anatomic landmarks that are identified on the ultrasound data and on the patient's joint, e.g. a trochlea, a cartilage shape or a subchondral bone shape.

The use of a holding apparatus can be advantageous, but is not necessary for obtaining 3D ultrasound images. Alternatively, 3D ultrasound images can be obtained, for example, by sweeping or moving the transducer during the acquisition.

The ultrasound images can be used to obtain information about at least one of: Joint dimensions, e.g. AP, ML, SI or in oblique planes; Joint curvature, e.g. cartilage or subchondral bone; Osteophyte shape; Subchondral cysts; Subchondral sclerosis; Slopes, e.g. tibial slopes.

Optionally, the ultrasound images, 2D or 3D, can be obtained during joint motion. In this manner, the relative motion of a first articular surface relative to a second articular surface can be captured. 4D imaging is a preferred mode for imaging of joint motion, with the three dimensions being space and the 4^{th} dimension being time or motion.

Joint motion that can be measured can include, but is not limited to: Translation of one articular surface relative to the other; Rotation of one articular surface relative to the other; any of the measurement can be done during: Flexion; Extension; Abduction; Adduction; Elevation; Internal rotation; External rotation; And other joint movements.

If a holding apparatus is used for the transducer(s), it can optionally include one or more holding apparatus joints so that the holding apparatus does not restrict joint motion, but allows for movement of the transducers with the patient's joint.

One or more transducers can be mounted to the holding apparatus. For example, in a knee joint, a first transducer can include the femur and, optionally, portions of the tibia, a second transducer can include the tibia and, optionally, portions of the femur, and a third transducer can include the patella and, optionally, portions of the femur.

The overlap between opposing articular surfaces in the field of view covered by the one or more transducers allows for accurate registration of the articular surfaces during joint motion.

The movement of the holding apparatus joints or the holding apparatus can be mechanically or electronically captured (over time) along multiple degrees of freedom and, for example, using a time stamp or reference, can be referenced to the ultrasound images obtained during joint motion. The motion can, for example, be captured using a gyroscope or mechanical means. In this manner, the position of the ultrasound scanners relative to the limb or a bone can be captured during joint motion and can be referenced to the images.

The resultant kinematic scan data (3D or 4D) can be used to assess joint motion prior to surgery. Such ultrasound based kinematic data can be captured for the joint that will be operated or for the contralateral joint.

A surgical procedure, e.g. a ligament repair (e.g. ACL), an osteotomy or an implant placement can then be simulated on the data. If an implant placement is performed, optionally virtual cuts, drilling or reaming can be introduced. The implant surfaces can be superimposed and the kinematics or biomotion after implant placement can be assessed and compared to the unoperated state.

Many simulations and optimizations that can be performed in order to achieve postoperative kinematics that closely resemble the preoperative kinematics or in the case of severe arthritis that resemble the kinematics of the patient in the pre-arthritic state. These simulations or optimizations can include: Selection of an implant size; Selection of implant shape(s), e.g. on a femur or a tibia or a tibial insert shape (including, for example, sagittal curvature, coronal curvature of femoral component(s), tibial component, insert height etc.); Selection of an implant position; Selection of an implant orientation; Selection of a resection height or level, e.g. on a femur or a tibia or a glenoid or an acetabulum or a femoral neck in order to maintain a joint line location after implantation similar to the unoperated state.

If a patient specific implant is used, any of the following parameters can be adapted or changed in order to optimize the kinematic result relative to the preoperative simulation (based on ultrasound, other scans or databases or combinations thereof):

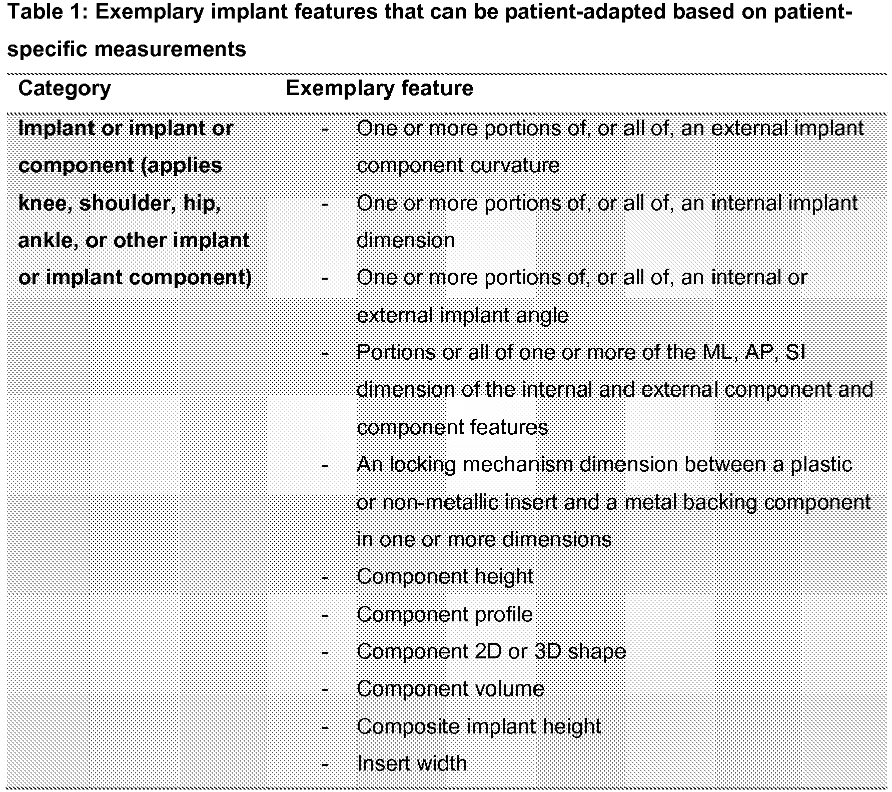

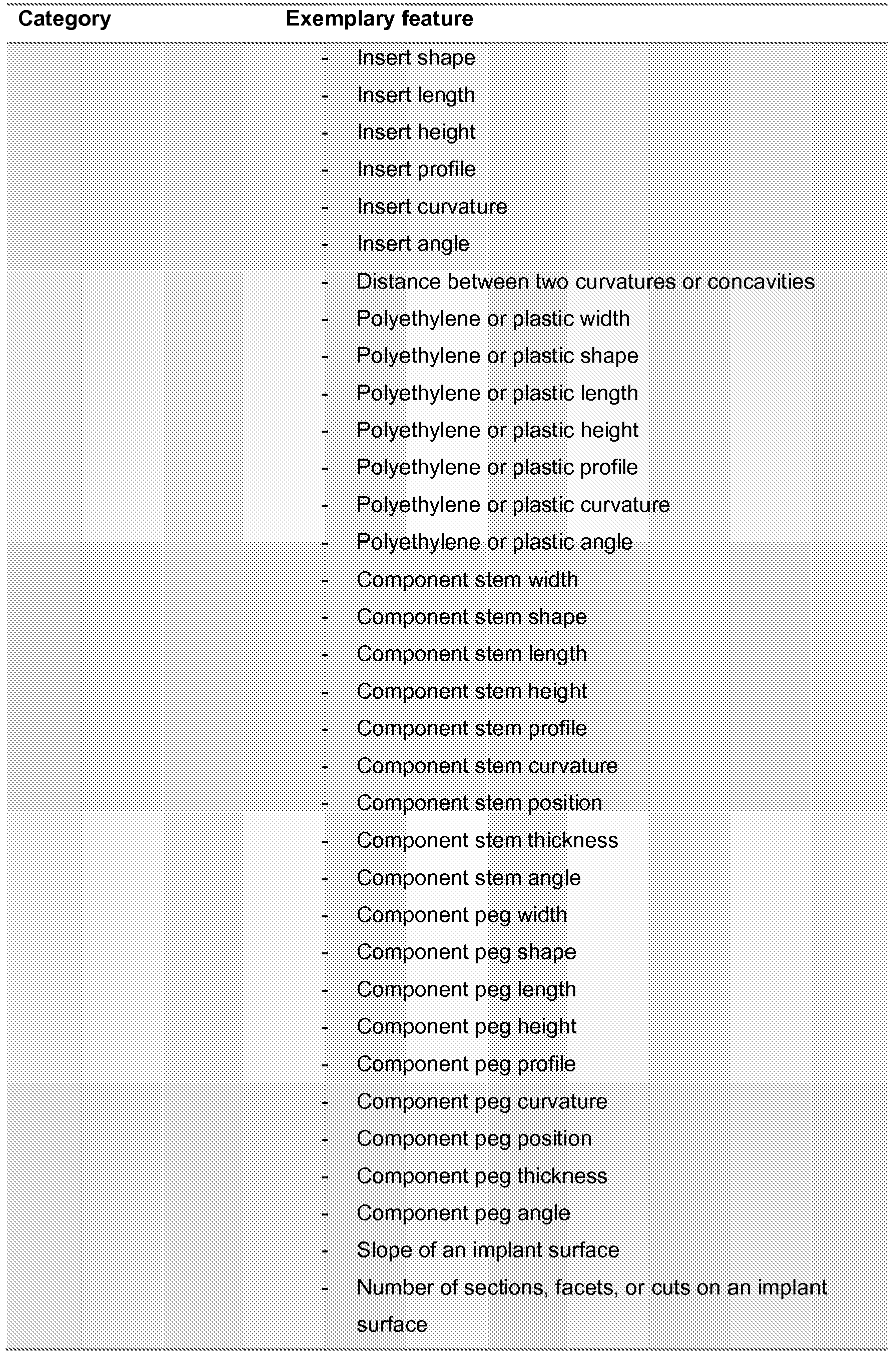

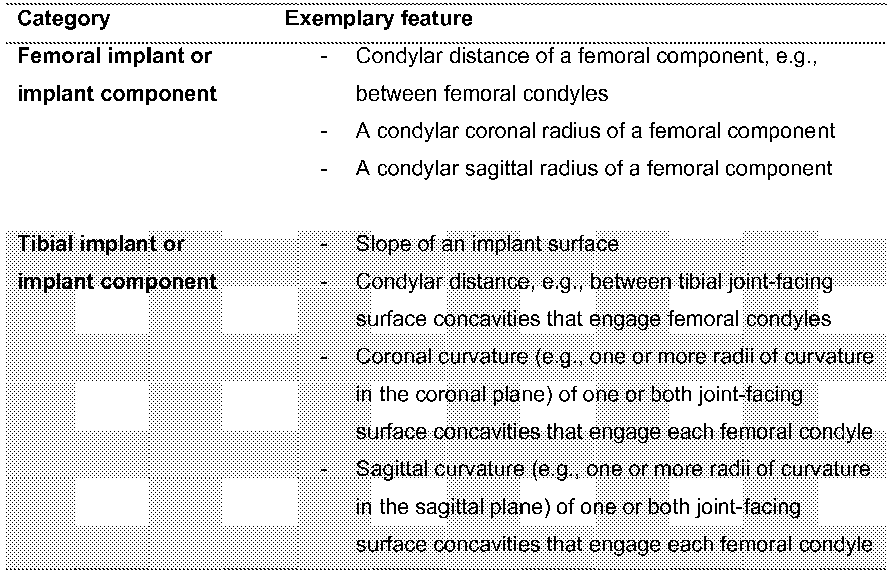

The patient-adapted features described in **Table 1** also can be applied to patient-adapted guide tools described herein.

### H. Establishing Normal or Near-Normal Joint Kinematics

In certain embodiments, bone cuts and implant shape including at least one of a bone-facing or a joint-facing surface of the implant can be designed or selected to achieve normal joint kinematics.

In certain embodiments, a computer program simulating biomotion of one or more joints, such as, for example, a knee joint, or a knee and ankle joint, or a hip, knee and/or ankle joint can be utilized. In certain embodiments, patient-specific imaging data can be fed into this computer program. For example, a series of two-dimensional images of a patient's knee joint or a three-dimensional representation of a patient's knee joint can be entered into the program. Additionally, two-dimensional images or a three-dimensional representation of the patient's ankle joint and/or hip joint may be added.

Alternatively, patient-specific kinematic data, for example obtained in a gait lab, can be fed into the computer program. Alternatively, patient-specific navigation data, for example generated using a surgical navigation system, image guided or non-image guided can be fed into the computer program. This kinematic or navigation data can, for example, be generated by applying optical or RF markers to the limb and by registering the markers and then measuring limb movements, for example, flexion, extension, abduction, adduction, rotation, and other limb movements.

Optionally, other data including anthropometric data may be added for each patient. These data can include but are not limited to the patient's age, gender, weight, height, size, body mass index, and race. Desired limb alignment and/or deformity correction can be added into the model. The position of bone cuts on one or more articular surfaces as well as the intended location of implant bearing surfaces on one or more articular surfaces can be entered into the model.

A patient-specific biomotion model can be derived that includes combinations of parameters listed above. The biomotion model can simulate various activities of daily life including normal gait, stair climbing, descending stairs, running, kneeling, squatting, sitting and any other physical activity. The biomotion model can start out with standardized activities, typically derived from reference databases. These reference databases can be, for example, generated using biomotion measurements using force plates and motion trackers using radiofrequency or optical markers and video equipment.

The biomotion model can then be individualized with use of patient-specific information including at least one of, but not limited to the patient's age, gender, weight, height, body mass index, and race, the desired limb alignment or deformity correction, and the patient's imaging data, for example, a series of two-dimensional images or a three-dimensional representation of the joint for which surgery is contemplated.

An implant shape including associated bone cuts generated in the preceding optimizations, for example, limb alignment, deformity correction, bone preservation on one or more articular surfaces, can be introduced into the model. Table 2 includes an exemplary list of parameters that can be measured in a patient-specific biomotion model.

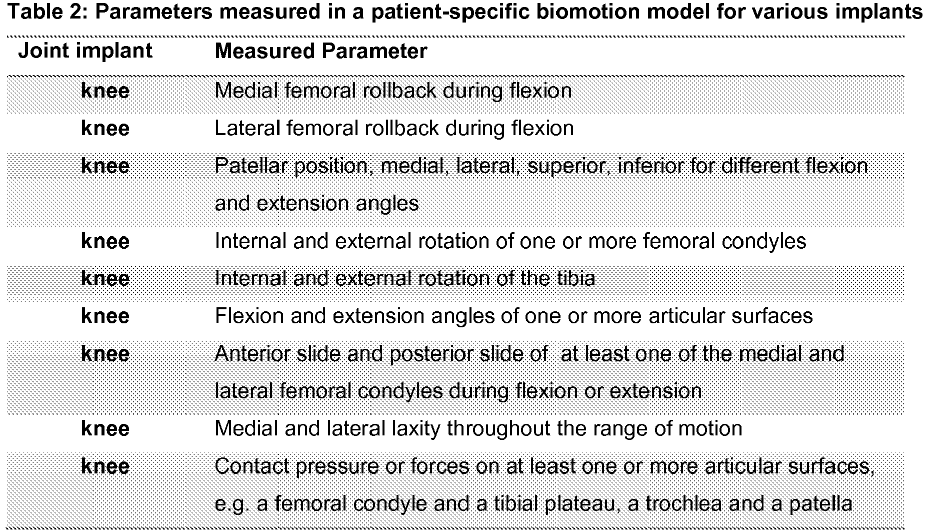

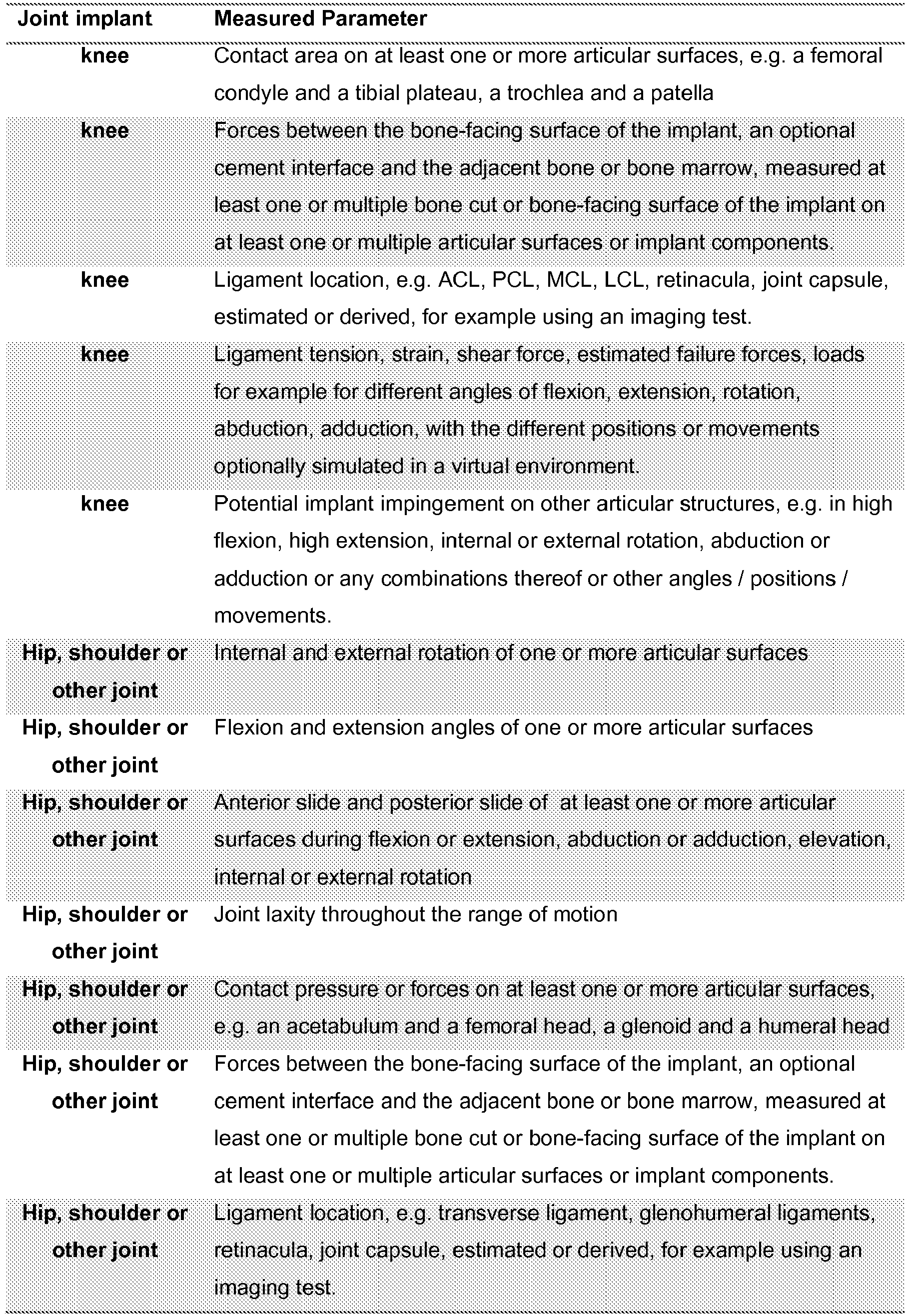

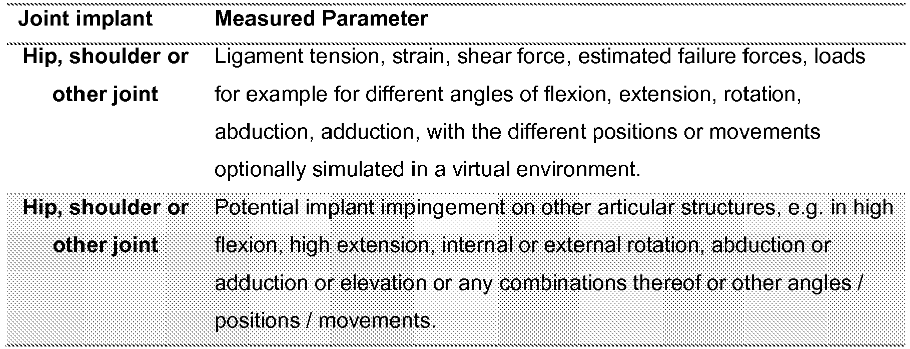

The above list is not meant to be exhaustive, but only exemplary. Any other biomechanical parameter known in the art can be included in the analysis.

The resultant biomotion data can be used to further optimize the implant design with the objective to establish normal or near normal kinematics. The implant optimizations can include one or multiple implant components. Implant optimizations based on patient-specific data including image based biomotion data include, but are not limited to: Changes to external, joint-facing implant shape in coronal plane; Changes to external, joint-facing implant shape in sagittal plane; Changes to external, joint-facing implant shape in axial plane; Changes to external, joint-facing implant shape in multiple planes or three dimensions; Changes to internal, bone-facing implant shape in coronal plane; Changes to internal, bone-facing implant shape in sagittal plane; Changes to internal, bone-facing implant shape in axial plane; Changes to internal, bone-facing implant shape in multiple planes or three dimensions; Changes to one or more bone cuts, for example with regard to depth of cut, orientation of cut; Any single one or combinations of the above or all of the above on at least one articular surface or implant component or multiple articular surfaces or implant components.

When changes are made on multiple articular surfaces or implant components, these can be made in reference to or linked to each other. For example, in the knee, a change made to a femoral bone cut based on patient-specific biomotion data can be referenced to or linked with a concomitant change to a bone cut on an opposing tibial surface, for example, if less femoral bone is resected, the computer program may elect to resect more tibial bone.

Similarly, if a femoral implant shape is changed, for example on an external surface, this can be accompanied by a change in the tibial component shape. This is, for example, particularly applicable when at least portions of the tibial bearing surface negatively-match the femoral joint-facing surface.

Similarly, if the footprint of a femoral implant is broadened, this can be accompanied by a widening of the bearing surface of a tibial component. Similarly, if a tibial implant shape is changed, for example on an external surface, this can be accompanied by a change in the femoral component shape. This is, for example, particularly applicable when at least portions of the femoral bearing surface negatively-match the tibial joint-facing surface.

Similarly, if a patellar component radius is widened, this can be accompanied by a widening of an opposing trochlear bearing surface radius, or vice-versa.

These linked changes also can apply for hip and/or shoulder implants. For example, in a hip, if a femoral implant shape is changed, for example on an external surface, this can be accompanied by a change in an acetabular component shape. This is, for example, applicable when at least portions of the acetabular bearing surface negatively-match the femoral joint-facing surface. In a shoulder, if a glenoid implant shape is changed, for example on an external surface, this can be accompanied by a change in a humeral component shape. This is, for example, particularly applicable when at least portions of the humeral bearing surface negatively-match the glenoid joint-facing surface, or vice-versa.

Any combination is possible as it pertains to the shape, orientation, and size of implant components on two or more opposing surfaces.

By optimizing implant shape in this manner, it is possible to establish normal or near normal kinematics. Moreover, it is possible to avoid implant related complications, including but not limited to anterior notching, notch impingement, posterior femoral component impingement in high flexion, and other complications associated with existing implant designs. For example, certain designs of the femoral components of traditional knee implants have attempted to address limitations associated with traditional knee implants in high flexion by altering the thickness of the distal and/or posterior condyles of the femoral implant component or by altering the height of the posterior condyles of the femoral implant component. Since such traditional implants follow a one-size-fits-all approach, they are limited to altering only one or two aspects of an implant design. However, with the design approaches described herein, various features of an implant component can be designed for an individual to address multiple issues, including issues associated with high flexion motion. For example, designs as described herein can alter an implant component's bone-facing surface (for example, number, angle, and orientation of bone cuts), joint-facing surface (for example, surface contour and curvatures) and other features (for example, implant height, width, and other features) to address issues with high flexion together with other issues.

Biomotion models for a particular patient can be supplemented with patient-specific finite element modeling or other biomechanical models known in the art. Resultant forces in the knee joint can be calculated for each component for each specific patient. The implant can be engineered to the patient's load and force demands. For instance, a 125lb. patient may not need a tibial plateau as thick as a patient with 280 lbs. Similarly, the polyethylene can be adjusted in shape, thickness and material properties for each patient. For example, a 3 mm polyethylene insert can be used in a light patient with low force and a heavier or more active patient may need an 8mm polymer insert or similar device.

Referring back to **FIG. 25****,** the variable nature of the interior piece facilitates obtaining the most accurate cut despite the level of disease of the joint because it positions the exterior piece ***2320*** such that it can achieve a cut that is perpendicular to the mechanical axis. Either the interior piece ***2310*** or the exterior piece ***2320*** can be formed out of any of the materials discussed above in Section II, or any other suitable material. Additionally, a person of skill in the art will appreciate that the invention is not limited to the two piece configuration described herein. The reusable exterior piece ***2320*** and the patient specific interior piece ***2310*** can be a single piece that is either patient specific (where manufacturing costs of materials support such a product) or is reusable based on a library of substantially defect conforming shapes developed in response to known or common tibial surface sizes and defects. The interior piece ***2310*** is typically molded to the tibia including the subchondral bone and/or the cartilage. The surgeon will typically remove any residual meniscal tissue prior to applying the mold. Optionally, the interior surface ***2312*** of the mold can include shape information of portions or all of the menisci.

Turning now to **FIG. 25B-D****,** a variety of views of the removable exterior piece ***2320.*** The top surface ***2322*** of the exterior piece can be relatively flat. The lower surface ***2324*** which abuts the interior piece conforms to the shape of the upper surface of the interior piece. In this illustration the upper surface of the interior piece is flat, therefore the lower surface ***2324*** of the reusable exterior surface is also flat to provide an optimal mating surface.

A guide plate ***2326*** is provided that extends along the side of at least a portion of the exterior piece ***2320.*** The guide plate ***2326*** provides one or more slots or guides ***2328*** through which a saw blade can be inserted to achieve the cut desired of the tibial surface. Additionally, the slot, or guide, can be configured so that the saw blade cuts at a line perpendicular to the mechanical axis, or so that it cuts at a line that is perpendicular to the mechanical axis, but has a 4-7 degree slope in the sagittal plane to match the normal slope of the tibia.

Optionally, a central bore ***2330*** can be provided that, for example, enables a drill to ream a hole into the bone for the stem of the tibial component of the knee implant.

**FIGS. 25E-H** illustrate the interior, patient specific, piece ***2310*** from a variety of perspectives. **FIG. 25E** shows a side view of the piece showing the uniform superior surface ***2314*** and the uniform side surfaces ***2316*** along with the irregular inferior surface ***2316.*** The inferior surface mates with the irregular surface of the tibia ***2302.*** **FIG. 25F** illustrates a superior view of the interior, patient, specific piece of the mold ***2310.*** Optionally having an aperture ***2330.*** **FIG. 25G** illustrates an inferior view of the interior patient specific mold piece ***2310*** further illustrating the irregular surface which includes convex and concave portions to the surface, as necessary to achieve optimal mating with the surface of the tibia.

**FIG. 25H** illustrates cross-sectional views of the interior patient specific mold piece ***2310.*** As can be seen in the cross-sections, the surface of the interior surface changes along its length.

As is evident from the views shown in **FIG. 25B** and **D****,** the length of the guide plate ***2326*** can be such that it extends along all or part of the tibial plateau, e.g. where the guide plate ***2326*** is asymmetrically positioned as shown in **FIG. 25B** or symmetrical as in **FIG. 23D****.** If total knee arthroplasty is contemplated, the length of the guide plate **2326** typically extends along all of the tibial plateau. If unicompartmental arthroplasty is contemplated, the length of the guide plate typically extends along the length of the compartment that the surgeon will operate on. Similarly, if total knee arthroplasty is contemplated, the length of the molded, interior piece ***2310*** typically extends along all of the tibial plateau; it can include one or both tibial spines. If unicompartmental arthroplasty is contemplated, the length of the molded interior piece typically extends along the length of the compartment that the surgeon will operate on; it can optionally include a tibial spine.

Turning now to **FIG. 25I****,** an alternative embodiment is depicted of the aperture ***2330.*** In this embodiment, the aperture features lateral protrusions to accommodate using a reamer or punch to create an opening in the bone that accepts a stem having flanges.

**FIGS. 25J** and **M** depict alternative embodiments designed to control the movement and rotation of the cutting block ***2320*** relative to the mold ***2310.*** As shown in **FIG. 25J** a series of protrusions, illustrated as pegs ***2340,*** are provided that extend from the superior surface of the mold. As will be appreciated by those of skill in the art, one or more pegs or protrusions can be used without departing from the scope of the invention. For purposes of illustration, two pegs have been shown in **FIG. 25J****.** Depending on the control desired, the pegs ***2340*** are configured to fit within, for example, a curved slot ***2342*** that enables rotational adjustment as illustrated in **FIG. 23K** or within a recess ***2344*** that conforms in shape to the peg ***2340*** as shown in **FIG. 25L****.** As will be appreciated by those of skill in the art, the recess ***2344*** can be sized to snugly encompass the peg or can be sized larger than the peg to allow limited lateral and rotational movement. The recess can be composed of a metal or other hard insert 544.

As illustrated in **FIG. 25M** the surface of the mold ***2310*** can be configured such that the upper surface forms a convex dome ***2350*** that fits within a concave well ***2352*** provided on the interior surface of the cutting block ***2320.*** This configuration enables greater rotational movement about the mechanical axis while limiting lateral movement or translation.

Other embodiments and configurations could be used to achieve these results without departing from the scope of the invention.

As will be appreciated by those of skill in the art, more than two pieces can be used, where appropriate, to comprise the system. For example, the patient specific interior piece ***2310*** can be two pieces that are configured to form a single piece when placed on the tibia. Additionally, the exterior piece ***2320*** can be two components. The first component can have, for example, the cutting guide apertures ***2328.*** After the resection using the cutting guide aperture ***2328*** is made, the exterior piece ***2320*** can be removed and a secondary exterior piece ***2320'*** can be used which does not have the guide plate **2326** with the cutting guide apertures ***2328,*** but has the aperture ***2330*** which facilitates boring into the tibial surface an aperture to receive a stem of the tibial component of the knee implant. Any of these designs could also feature the surface configurations shown in **FIGS. 25J-M****,** if desired.

**FIG. 25N** illustrates an alternative design of the cutting block ***2320*** that provides additional structures ***2360*** to protect, for example, the cruciate ligaments, from being cut during the preparation of the tibial plateau. These additional structures can be in the form of indented guides ***2360,*** as shown in **FIG. 25N** or other suitable structures.

**FIG. 25O** illustrates a cross-section of a system having anchoring pegs **2362** on the surface of the interior piece ***2310*** that anchor the interior piece ***2310*** into the cartilage or meniscal area.

**FIGS. 25P** AND **Q** illustrate a device ***2300*** configured to cover half of a tibial plateau such that it is unicompartmental.

**FIG. 25R** illustrates an interior piece **2310** that has multiple contact surfaces **2312** with the tibial **2302,** in accordance with one embodiment. As opposed to one large contact surface, the interior piece **2310** includes a plurality of smaller contact surfaces **2312.** In various embodiments, the multiple contact surfaces **2312** are not on the sample plane and are at angles relative to each other to ensure proper positioning on the tibia **2302.** Two or three contact surfaces **2312** may be required to ensure proper positioning. In various embodiments, only the contact surfaces **2312** of the interior piece may be molded, the molds attached to the rest of the template using methodologies known in the art, such as adhesives. The molds may be removably attached to the template. It is to be understood that multiple contact surfaces **2312** may be utilized in template embodiments that include one or a plurality of pieces.

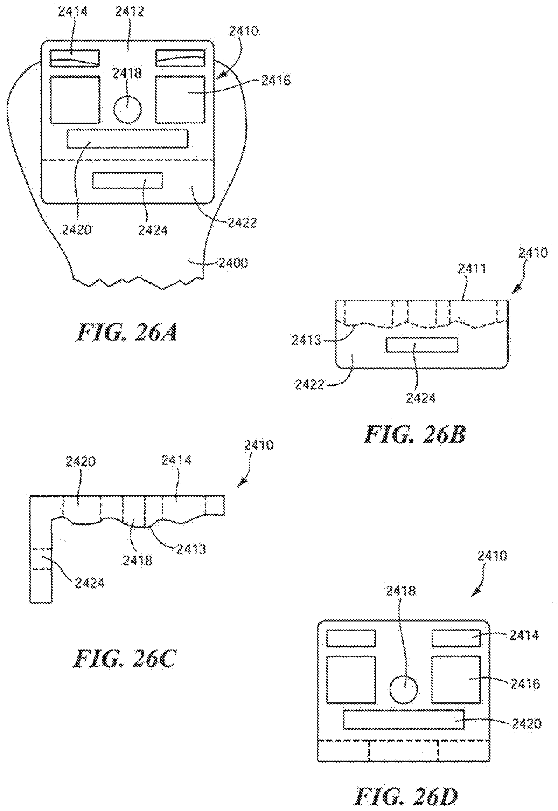

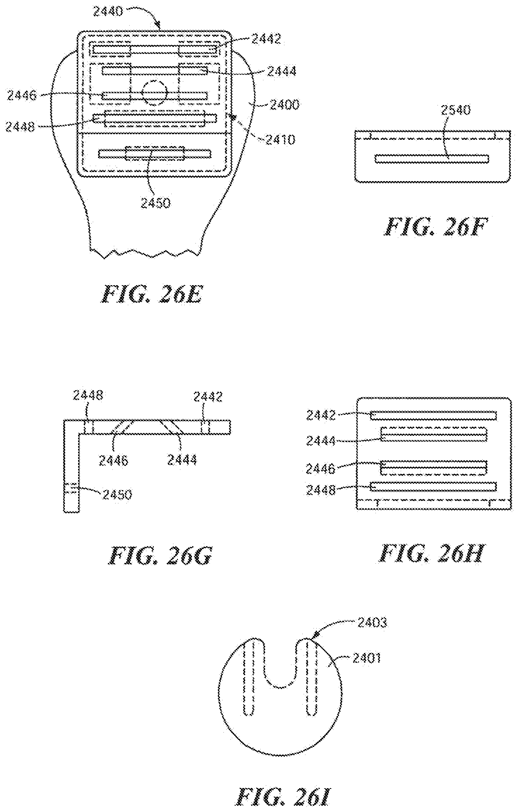

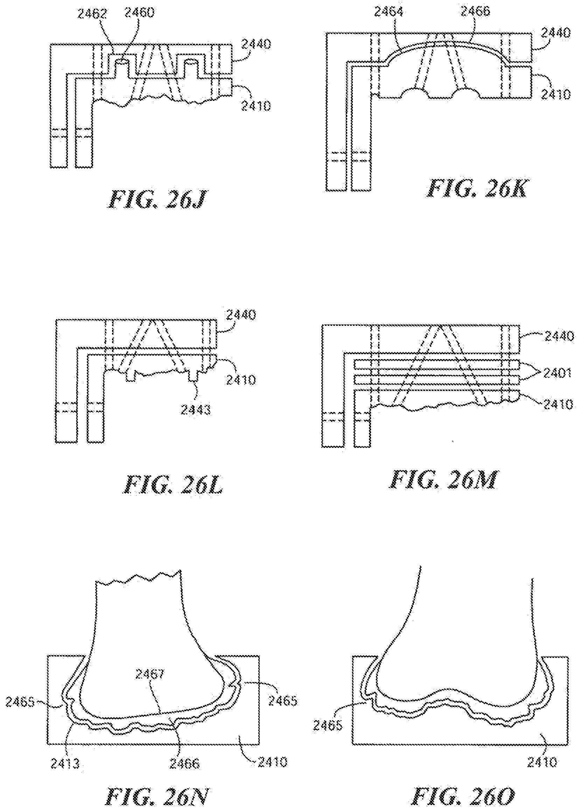

Turning now to **FIG. 26****,** a femoral mold system is depicted that facilitates preparing the surface of the femur such that the finally implanted femoral implant will achieve optimal mechanical and anatomical axis alignment.

**FIG. 26A** illustrates the femur ***2400*** with a first portion ***2410*** of the mold placed thereon. In this depiction, the top surface of the mold ***2412*** is provided with a plurality of apertures. In this instance the apertures consist of a pair of rectangular apertures ***2414,*** a pair of square apertures ***2416,*** a central bore aperture ***2418*** and a long rectangular aperture ***2420.*** The side surface ***2422*** of the first portion ***2410*** also has a rectangular aperture ***2424.*** Each of the apertures is larger than the eventual cuts to be made on the femur so that, in the event the material the first portion of the mold is manufactured from a soft material, such as plastic, it will not be inadvertently cut during the joint surface preparation process. Additionally, the shapes can be adjusted, *e.g.,* rectangular shapes made trapezoidal, to give a greater flexibility to the cut length along one area, without increasing flexibility in another area. As will be appreciated by those of skill in the art, other shapes for the apertures, or orifices, can be changed without departing from the scope of the invention.

**FIG. 26B** illustrates a side view of the first portion ***2410*** from the perspective of the side surface ***2422*** illustrating the aperture ***2424.*** As illustrated, the exterior surface ***2411*** has a uniform surface which is flat, or relatively flat configuration while the interior surface ***2413*** has an irregular surface that conforms, or substantially conforms, with the surface of the femur.

**FIG 26C** illustrates another side view of the first, patient specific molded, portion ***2410,*** more particularly illustrating the irregular surface ***2413*** of the interior. **FIG. 26D** illustrates the first portion ***2410*** from a top view. The center bore aperture ***2418*** is optionally provided to facilitate positioning the first piece and to prevent central rotation.

**FIG. 26D** illustrates a top view of the first portion ***2410.*** The bottom of the illustration corresponds to an anterior location relative to the knee joint. From the top view, each of the apertures is illustrated as described above. As will be appreciated by those of skill in the art, the apertures can be shaped differently without departing from the scope of the invention.

Turning now to **FIG. 26E****,** the femur ***2400*** with a first portion ***2410*** of the cutting block placed on the femur and a second, exterior, portion ***2440*** placed over the first portion ***2410*** is illustrated. The second, exterior, portion ***2440*** features a series of rectangular grooves ***(2442-2450)*** that facilitate inserting a saw blade therethrough to make the cuts necessary to achieve the femur shape illustrated in **FIG. 21E****.** These grooves can enable the blade to access at a 90° angle to the surface of the exterior portion, or, for example, at a 45° angle. Other angles are also possible without departing from the scope of the invention.

As shown by the dashed lines, the grooves **(*2442-2450*)** of the second portion ***2440,*** overlay the apertures of the first layer.

**FIG. 26F** illustrates a side view of the second, exterior, cutting block portion ***2440.*** From the side view a single aperture ***2450*** is provided to access the femur cut. **FIG. 26G** is another side view of the second, exterior, portion ***2440*** showing the location and relative angles of the rectangular grooves. As evidenced from this view, the orientation of the grooves ***2442, 2448*** and ***2450*** is perpendicular to at least one surface of the second, exterior, portion ***2440.*** The orientation of the grooves ***2444, 2446*** is at an angle that is not perpendicular to at least one surface of the second, exterior portion ***2440.*** These grooves **(*2444, 2446*)** facilitate making the angled chamfer cuts to the femur. **FIG. 26H** is a top view of the second, exterior portion ***2440.*** As will be appreciated by those of skill in the art, the location and orientation of the grooves will change depending upon the design of the femoral implant and the shape required of the femur to communicate with the implant.

**FIG.26I** illustrates a spacer ***2401*** for use between the first portion ***2410*** and the second portion ***2440.*** The spacer ***2401*** raises the second portion relative to the first portion, thus raising the area at which the cut through groove ***2424*** is made relative to the surface of the femur. As will be appreciated by those of skill in the art, more than one spacer can be employed without departing from the scope of the invention. Spacers can also be used for making the tibial cuts. Optional grooves or channels ***2403*** can be provided to accommodate, for example, pins ***2460*** shown in **FIG. 26J****.**

Similar to the designs discussed above with respect to **FIG. 25****,** alternative designs can be used to control the movement and rotation of the cutting block ***2440*** relative to the mold ***2410.*** As shown in **FIG. 26J** a series of protrusions, illustrated as pegs ***2460,*** are provided that extend from the superior surface of the mold. These pegs or protrusions can be telescoping to facilitate the use of molds if necessary. As will be appreciated by those of skill in the art, one or more pegs or protrusions can be used without departing from the scope of the invention. For purposes of illustration, two pegs have been shown in **FIG. 26J****.** Depending on the control desired, the pegs ***2460*** are configured to fit within, for example, a curved slot that enables rotational adjustment similar to the slots illustrated in **FIG. 25K** or within a recess that conforms in shape to the peg, similar to that shown in **FIG. 25L** and described with respect to the tibial cutting system. As will be appreciated by those of skill in the art, the recess ***2462*** can be sized to snugly encompass the peg or can be sized larger than the peg to allow limited lateral and rotational movement.

As illustrated in **FIG. 26K** the surface of the mold ***2410*** can be configured such that the upper surface forms a convex dome ***2464*** that fits within a concave well ***2466*** provided on the interior surface of the cutting block ***2440.*** This configuration enables greater rotational movement about the mechanical axis while limiting lateral movement or translation.

In installing an implant, first the tibial surface is cut using a tibial block, such as those shown in **FIG. 26****.** The patient specific mold is placed on the femur. The knee is then placed in extension and spacers ***2401,*** such as those shown in **FIG. 26M****,** or shims are used, if required, until the joint optimal function is achieved in both extension and flexion. The spacers, or shims, are typically of an incremental size, *e.g.,* 5 mm thick to provide increasing distance as the leg is placed in extension and flexion. A tensiometer can be used to assist in this determination or can be incorporated into the mold or spacers in order to provide optimal results. The design of tensiometers are known in the art, including, for example, those described in U.S. Patent 5,630,820 to Todd issued May 20, 1997.
As illustrated in **FIGS. 26N** (sagittal view) and **26O** (coronal view), the interior surface ***2413*** of the mold ***2410*** can include small teeth ***2465*** or extensions that can help stabilize the mold against the cartilage ***2466*** or subchondral bone ***2467.***

### I. Hip Joint

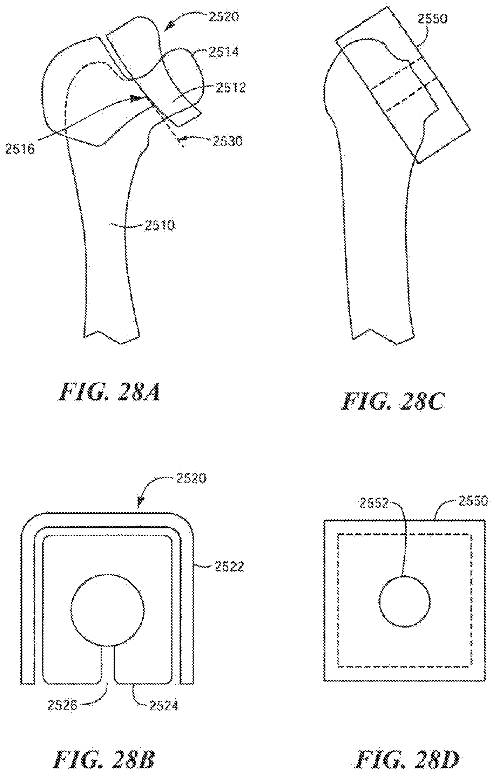

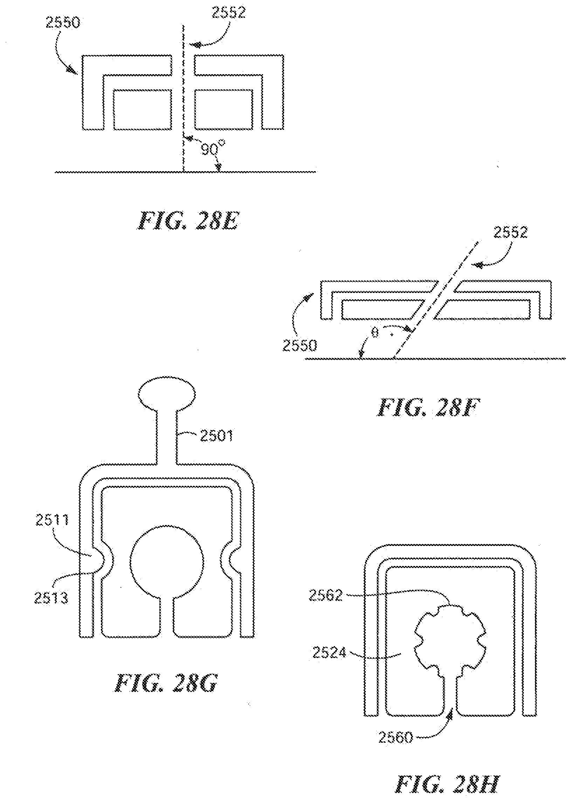

Turning now to **FIG. 28****,** a variety of views showing sample mold and cutting block systems for use in the hip joint are shown. **FIG. 28A** illustrates femur ***2510*** with a mold and cutting block system **2520** placed to provide a cutting plane ***2530*** across the femoral neck ***2512*** to facilitate removal of the head ***2514*** of the femur and creation of a surface ***2516*** for the hip ball prosthesis.

**FIG. 28B** illustrates a top view of the cutting block system 2520. The cutting block system 2520 includes an interior, patient specific, molded section 2524 and an exterior cutting block surface 2522. The interior, patient specific, molded section 2524 can include a canal 2526 to facilitate placing the interior section 2524 over the neck of the femur. As will be appreciated by those of skill in the art, the width of the canal will vary depending upon the rigidity of the material used to make the interior molded section. The exterior cutting block surface 2522 is configured to fit snugly around the interior section. Additional structures can be provided, similar to those described above with respect to the knee cutting block system, that control movement of the exterior cutting block 2524 relative to interior mold section 2522, as will be appreciated by those of skill in the art. Where the interior section 2524 encompasses all or part of the femoral neck, the cutting block system can be configured such that it aids in removal of the femoral head once the cut has been made by, for example, providing a handle 2501.

**FIG. 28C** illustrates a second cutting block system 2550 that can be placed over the cut femur to provide a guide for reaming after the femoral head has been removed using the cutting block shown in **FIG. 28A. FIG. 28D** is a top view of the cutting block shown in **FIG. 28C****.** As will be appreciated by those of skill in the art, the cutting block shown in **FIG. 28C-D****,** can be one or more pieces. As shown in **FIG. 28E****,** the aperture 2552 can be configured such that it enables the reaming for the post of the implant to be at a 90° angle relative to the surface of femur. Alternatively, as shown in **FIG. 28F****,** the aperture 2552 can be configured to provide an angle other than 90° for reaming, if desired.

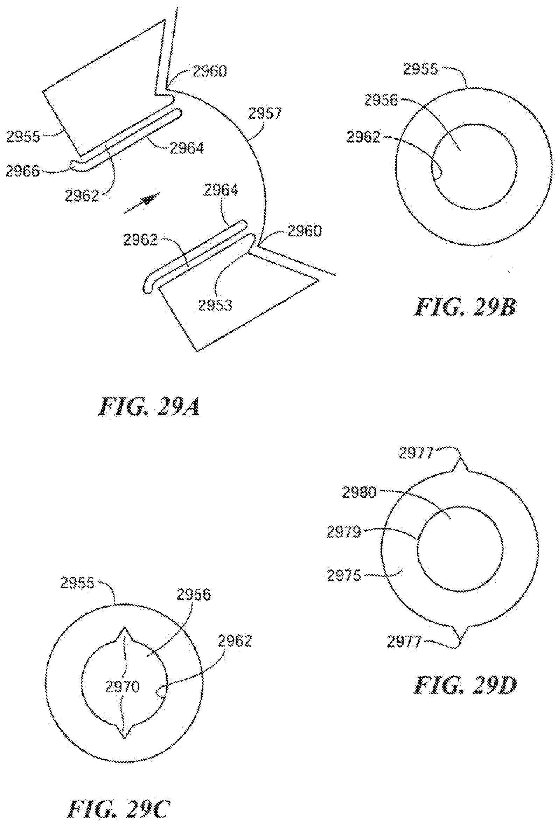

**FIGS. 29A** (sagittal view) and **29B** (frontal view, down onto mold) illustrates a mold system 2955 for the acetabulum 2957. The mold can have grooves 2959 that stabilize it against the acetabular rim 2960. Surgical instruments, e.g. reamers, can be passed through an opening in the mold 2956. The side wall of the opening 2962 can guide the direction of the reamer or other surgical instruments. Metal sleeves 2964 can be inserted into the side wall 2962 thereby protecting the side wall of the mold from damage.

The metal sleeves 2964 can have lips 2966 or overhanging edges that secure the sleeve against the mold and help avoid movement of the sleeve against the articular surface.

**FIG. 29C** is a frontal view of the same mold system shown in **FIGS. 29A** and **29B****.** A groove 2970 has been added at the 6 and 12 o'clock positions. The groove can be used for accurate positioning or placement of surgical instruments. Moreover, the groove can be useful for accurate placement of the acetabular component without rotational error. Someone skilled in the art will recognize that more than one groove or internal guide can be used in order to not only reduce rotational error but also error related to tilting of the implant. As seen **FIG 29D****,** the implant 2975 can have little extensions 2977 matching the grooves thereby guiding the implant placement. The extensions 2977 can be a permanent part of the implant design or they can be detachable. Note metal rim 2979 and inner polyethylene cup2980 of the acetabular component.

**FIG. 29D** illustrates a cross-section of a system where the interior surface 2960 of the molded section 2924 has teeth 2962 or grooves to facilitate grasping the neck of the femur.

Various steps may be performed in order to design and make 3D guidance templates for hip implants, in accordance with one embodiment.

For example, in an initial step, a discrepancy in the length of the left leg and right leg may be determined, for example, in millimeters. Leg length discrepancy may be determined, for example, using standing x-rays, typically including the entire leg but also cross-sectional imaging modalities such as CT or MRI.

A CT scout scan may be utilized to estimate leg length. Alternatively, select image slices through the hip and ankle joint may be utilized to estimate leg length either using CT or MRI.

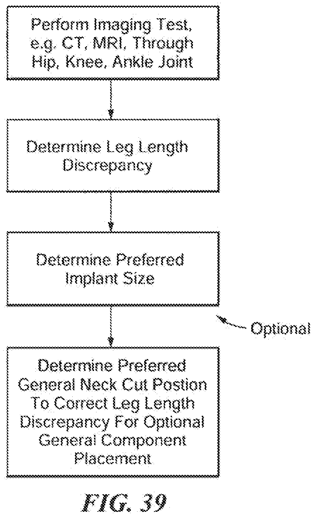

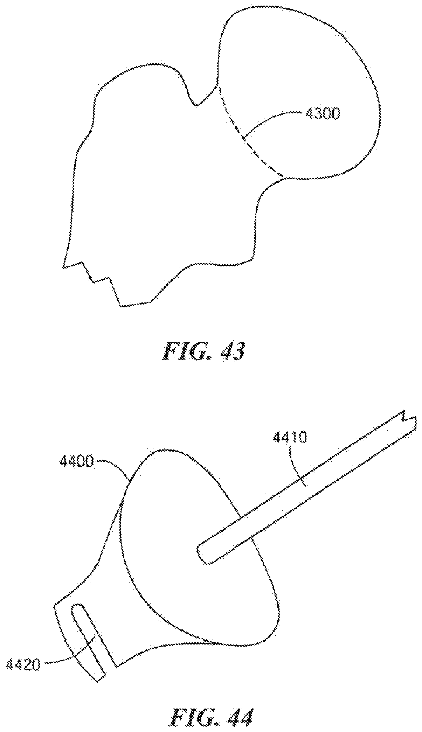

Pre-operative planning is then performed using the image data. A first 3D guidance template is designed to rest on the femoral neck. **FIG. 43** shows an example of an intended site 4300 for placement of a femoral neck mold for a total hip arthroplasty cut or saw plane integrated into this template, which can be derived. The position, shape and orientation of the 3D guidance mold or jig or template may be determined on the basis of anatomical axis such as the femoral neck axis, the biomechanical axis and/or also any underlying leg length discrepancy **(****FIG. 39****).** Specifically, the superoinferior cut or saw guide height can be adapted to account for leg length discrepancy. For example, if the left leg is five (5) millimeters shorter than the right leg, then the cut height can be moved by five (5) millimeters to account for this difference. The femoral neck cut height ultimately determines the position of the femoral stem. Thus, in this manner, using this type of pre-operative planning, the femoral neck cut height can be optimized using a 3D guidance template.

**FIG. 39** is a flow diagram of a method wherein measurement of leg length discrepancy can be utilized to determine the optimal cut height of the femoral neck cut for total hip arthroplasty. Initially, imaging is performed, e.g. CT and /or MRI, through, without limitation, the hip, knee and ankle joint, step 3902. Leg length discrepancy is determined, using the imaging data obtained, step 3904. The preferred implant size may then be optionally determined, step 3906. The preferred femoral neck cut position is determined based, at least in part, on correcting the leg length discrepancy for optimal femoral component placement.

**FIG. 44** shows another example of a femoral neck mold 4400 with handle 4410 and optional slot 4420.

### Acetabulum

In the acetabulum, the position and orientation of the acetabular component or acetabular cup is also critical for the success of hip surgery. For example, the lowest portion of the acetabular cup may be placed so that it is five (5) millimeters lateral to an anatomic landmark on a pelvic x-ray coinciding with the inferior border of the radiographic tear drop. If the acetabular component is, for example, placed too far superiorly, significant bone may be lost.

Placing the acetabular component using the 3D guidance template may include, for example, the following steps: Step One: Imaging, e.g. using optical imaging methods, CT or MRI; Step Two: Determining the anterior rotation of the acetabulum and the desired rotation of the acetabular cup; Step Three: Find best fitting cup size; Step Four: Determine optimal shape, orientation and/or position of 3D guidance template.

The template may be optionally designed to rest primarily on the margin of the acetabular fossa. In this manner, it is possible to ream through the template.

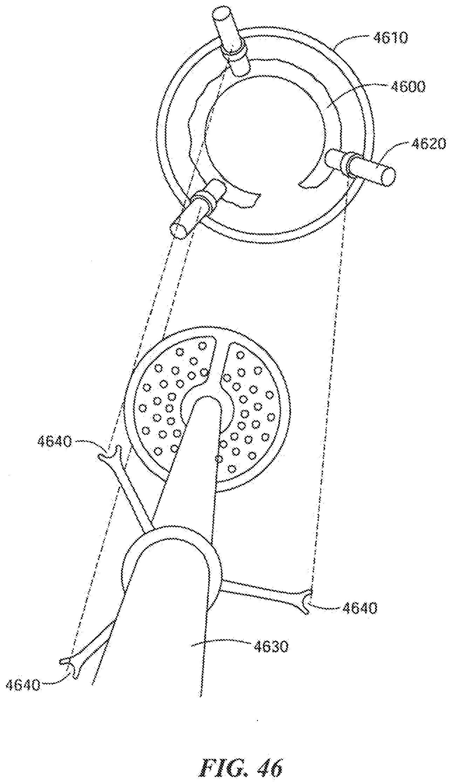

**FIG. 46** shows an example of a guidance mold used for reaming the site for an acetabular cup. The mold 4600 can be optionally attached to a generic frame 4610. A guide for the reamer is shown 4620. The reamer 4630 or the mold can have optional stops 4640. In this example, the stops 4640 are attached to the reamer 4630 and engage the guide 4620 for the reamer.

For purposes of reaming, the template may be fixed to the pelvis, for example, using metal spikes or K-wires. The template may also have a grip for fixing it to the bone. Thus, a surgeon may optionally press the template against the bone while a second surgeon will perform the reaming through the opening in the template. The grip or any stabilizers can extend laterally, and optionally serve as tissue retractors, keeping any potentially interfering soft tissue out of the surgical field. The template may also include stoppers 4640 to avoid over penetration of the reamer. These stoppers may be designed in the form of metal stops defining the deepest penetration area for the peripheral portion or other portions of the reamer. Optionally, the template may also taper and decrease in inner radius thereby creating a stop once the reamer once the reaches the innermost portion of the template. Any stop known in the art can be used. The imaging test can be used to design or shape the mold in a manner that will help achieve the optimal reaming depth. The stops can be placed on the mold or reamer in reference to the imaging test in order to achieve the optional reaming depth.

A 3D guidance template may be utilized to optimize the anteversion of the acetabular cup. For example, with the posteriolateral approach, typically an anteversion of forty to forty-five degrees is desired in both males and females. With an anterolateral approach, zero degrees anteversion may be desired. Irrespective of the desired degree of anti-version, the shape, orientation and/or position of the template may be optimized to include the desired degree of anteversion.

Similarly, on the femoral side, the 3D guidance template may be optimized with regard to its shape, orientation and position in order to account for neutral, varus or valgus position of the femoral shaft. A 3D guidance template may also be utilized to optimize femoral shaft anteversion.

Thus, after a first template has been utilized for performing the femoral neck cut and a second template has been utilized for performing the surgical intervention on the acetabular side, a third template may optionally be utilized to be placed onto the femoral cut.

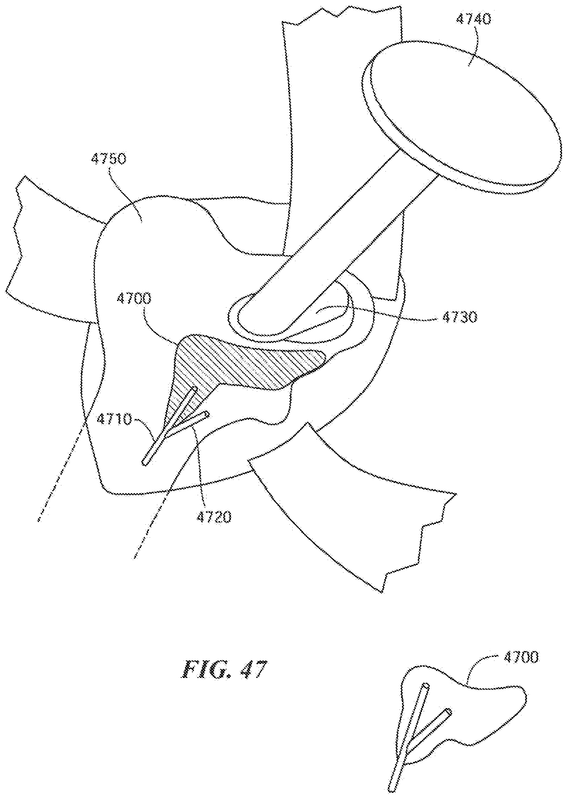

**FIG. 47** shows an example of an optional third mold 4700, placed on the femoral neck cut, providing an estimate of anteversion and longitudinal femoral axis.

The third template may optionally include a handle. The third template may be shaped, designed, oriented and/or positioned so that it is optimized to provide the surgeon with information and reference points for the long axis of the femur 4710 and femoral anteversion 4720. A broach 4730 with broach handle 4740 is seen in place. The cut femoral neck 4750 is seen. The third mold 4700 attaches to it. By providing information on the long axis of the femur and femoral anteversion, an intra-operative x-ray can be saved which would otherwise be necessitated in order to obtain this information.

Optionally, modular hip implant components may be utilized such as a modular stem. Such modular designs can be helpful in further optimizing the resultant femoral anteversion by selecting, for example, different stem shapes.

In another embodiment, the surgeon may perform a femur first technique wherein a first cut is applied to the femur using a first 3D guidance mold. Optionally, the broach in the cut femoral shaft may be left in place. Optionally, a trial implant head may be applied to the broach. The trial implant head may be variable in radius and superoinferior diameter and may be utilized to determine the optimal soft tissue tension. Optionally, the trial head may also be utilized to determine the acetabular cup position wherein said acetabular cup position is derived on the basis of the femoral cut. Thus, the acetabular position can be optionally derived using the opposite articular surface. In a reverse acetabulum first technique, the acetabulum can be prepared first and, using soft tissue balancing techniques, the femoral component can be placed in reference to the acetabular component. Optionally, the femoral cut may even be placed intentionally too proximal and is subsequently optimized by measuring soft tissue tension utilizing various trial heads with the option to then change the height of the optimal femoral cut.

The foregoing description of embodiments has been provided for the purposes of illustration and description. It is not intended to be exhaustive or to limit the invention to the precise forms disclosed. Many modifications and variations will be apparent to the practitioner skilled in the art. The embodiments were chosen and described in order to best explain the principles of the invention and its practical application, thereby enabling others skilled in the art to understand the invention and the various embodiments and with various modifications that are suited to the particular use contemplated.

## Claims

1. An articular repair system for performing a joint arthroplasty to repair a joint of a patient, the system comprising:
an implant configured to resurface or replace at least a portion of the joint of the patient; and
a patient-adapted surgical tool (2310) having a surface configured based on electronic image data of the joint of the patient, wherein the surface is configured to conform to and mate with a portion of a surface of the joint of the patient; **characterised in that** the system further comprises
one or more measurement devices configured for use in obtaining one or more kinematic measurements, wherein the one or more measurement devices are attached to or integrated into the patient-adapted surgical tool.

2. The system of claim 1, wherein the one or more measurement devices include an electronic sensor.

3. The system of claim 1, wherein the one or more measurement devices include a pressure sensor.

4. The system of claim 1, wherein the one or more measurement devices include a local contact pressure sensor.

5. The system of claim 4, wherein the implant comprises a tibial implant and the one or more implant components comprise one or more tibial inserts.

6. The system of claim 1, wherein the one or more measurement devices include markers, wherein the markers are optionally optoelectronic, radiofrequency or optical markers.

## Patentansprüche

1. Ein artikuläres Reparatursystem zur Durchführung einer Gelenk-Arthroplastik zur Reparatur eines Gelenks eines Patienten, das System umfassend:
ein Implantat konfiguriert, um mindestens einen Teil des Gelenks des Patienten mit einem neuen Belag zu versehen oder zu ersetzen; und
ein Patienten-adaptiertes chirurgisches Werkzeug (2310), das eine Oberfläche konfiguriert basierend auf elektronischen Bilddaten des Gelenks des Patienten hat, wobei die Oberfläche konfiguriert ist, um einem Teil einer Oberfläche des Gelenks des Patienten zu entsprechen und sich mit diesem zu verbinden; charakterisiert dadurch, dass das System ferner umfasst
ein oder mehrere Messinstrumente konfiguriert zur Verwendung bei der Gewinnung von einer oder mehreren kinematischen Messungen, wobei das eine oder mehrere Messinstrumente an das Patienten-spezifische Werkzeug befestigt oder darin integriert sind.

2. Das System nach Anspruch 1, wobei das eine oder mehrere Messinstrumente einen elektronischen Sensor beinhalten.

3. Das System nach Anspruch 1, wobei das eine oder mehrere Messinstrumente einen Drucksensor beinhalten.

4. Das System nach Anspruch 1, wobei das eine oder mehrere Messinstrumente einen lokalen Kontaktdrucksensor beinhalten.

5. Das System nach Anspruch 4, wobei das Implantat ein tibiales Implantat umfasst und die eine oder mehrere Implantat-Komponenten ein oder mehrere tibiale Inserts umfasst.

6. Das System nach Anspruch 1, wobei das eine oder mehrere Messinstrumente Marker beinhalten, wobei die Marker optional optoelektronische, Radiofrequenz- oder optische Marker sind.

## Revendications

1. Système de réparation articulaire pour effectuer une arthroplastie d'articulation pour réparer une articulation d'un patient, le système comprenant :
un implant configuré pour remodeler ou remplacer au moins une partie de l'articulation du patient ; et
un outil chirurgical adapté au patient (2310) ayant une surface configurée sur la base de données d'images électroniques de l'articulation du patient, dans lequel la surface est configurée pour épouser et correspondre à une partie d'une surface de l'articulation du patient ; **caractérisé en ce que** le système comprend en outre
un ou plusieurs dispositifs de mesure configurés pour être utilisés dans l'obtention d'une ou plusieurs mesures cinématiques, dans lequel les un ou plusieurs dispositifs de mesure sont fixés ou intégrés à l'outil chirurgical adapté au patient.

2. Système selon la revendication 1, dans lequel les un ou plusieurs dispositifs de mesure incluent un capteur électronique.

3. Système selon la revendication 1, dans lequel les un ou plusieurs dispositifs de mesure incluent un capteur de pression.

4. Système selon la revendication 1, dans lequel les un ou plusieurs dispositifs de mesure incluent un capteur de pression de contact locale.

5. Système selon la revendication 4, dans lequel l'implant comprend un implant tibial et les un ou plusieurs composants d'implant comprennent un ou plusieurs inserts tibiaux.

6. Système selon la revendication 1, dans lequel les un ou plusieurs dispositifs de mesure incluent des marqueurs, dans lequel les marqueurs sont éventuellement des marqueurs optoélectroniques, radiofréquence ou optiques.
